(19) Europäisches Patentamt
European Patent Office
Office européen des brevets

(11) **EP 4 810 479 A1**

(12) **EUROPEAN PATENT APPLICATION**
published in accordance with Art. 153(4) EPC

(43) Date of publication:
**23.09.2026 Bulletin 2026/39**

(21) Application number: **24891352.7**

(22) Date of filing: **11.11.2024**

(51) International Patent Classification (IPC):
***C07C 253/32*** *(2006.01)* ***C07C 39/06*** *(2006.01)*
***C07C 39/07*** *(2006.01)* ***C07C 39/08*** *(2006.01)*
***C07C 43/23*** *(2006.01)* ***C07C 69/732*** *(2006.01)*
***C07C 255/09*** *(2006.01)* ***C07C 255/23*** *(2006.01)*
***C07C 309/04*** *(2006.01)* ***C08F 2/40*** *(2006.01)*
***C08F 2/44*** *(2006.01)* ***C08F 22/30*** *(2006.01)*
***C08F 22/34*** *(2006.01)* ***C08K 5/13*** *(2006.01)*
***C08L 35/04*** *(2006.01)* ***C09J 4/00*** *(2006.01)*

(52) Cooperative Patent Classification (CPC):
**C07C 39/06; C07C 39/07; C07C 39/08; C07C 43/23; C07C 69/732; C07C 253/32; C07C 255/09; C07C 255/23; C07C 309/04; C08F 2/40; C08F 2/44; C08F 22/30; C08F 22/34; C08K 5/13; C08L 35/04;** (Cont.)

(86) International application number:
**PCT/JP2024/039898**

(87) International publication number:
**WO 2025/105316 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.11.2023 JP 2023193002**
**28.03.2024 JP 2024052780**

(71) Applicant: **Kuraray Co., Ltd.**
**Kurashiki-shi, Okayama 710-0801 (JP)**

(72) Inventors:
- **INATOMI Atsushi**
**Tsukuba-shi, Ibaraki 305-0841 (JP)**
- **SATO Hanaka**
**Tsukuba-shi, Ibaraki 305-0841 (JP)**
- **SHIROTA Kentaro**
**Tsukuba-shi, Ibaraki 305-0841 (JP)**

(74) Representative: **Müller-Boré & Partner Patentanwälte PartG mbB**
**Friedenheimer Brücke 21**
**80639 München (DE)**

(54) **1,1-DICYANOETHYLENE-CONTAINING COMPOSITION, CURED PRODUCT, AND LAMINATE**

(57) Disclosed is a 1,1-dicyanoethylene-containing composition containing 1,1-dicyanoethylene and an o-substituted phenol compound (A-1) represented by a specific formula (I). Also disclosed is a 1,1-dicyanoethylene-containing composition containing 1,1-dicyanoethylene, a phenol compound (A-2) represented by a specific formula (II), and a compound (C) having a proton affinity (Epa) of 780 kJ/mol or less and a pKa of 5 or less in the form of an aqueous solution.

EP 4 810 479 A1

(52) Cooperative Patent Classification (CPC): (Cont.)
**C09J 4/00**

## Description

Technical Field

The present invention relates to a 1,1-dicyanoethylene-containing composition, a cured product, and a laminate.

Background Art

Since 1,1-dicyanoethylene is excellent in reactivity, it is sometimes used for a curable adhesive.

However, due to its high reactivity, it is essential to use an acid stabilizer to enhance the storage stability in order to use 1,1-dicyanoethylene. PTLs 1 and 2 propose the use of benzenesulfonic acid, chlorobenzenesulfonic acid, p-toluenesulfonic acid, or the like as an acid stabilizer.

Methylenemalonic acid is a compound having high reactivity similar to 1,1-dicyanoethylene. PTL 3 proposes to enhance storage stability by allowing an antioxidant and an acid to coexist with methylenemalonic acid. In PTL 3, specifically, acetic acid is used as an acid in Examples.

Citation List

Patent Literature

PTL 1:US 2,665,298
PTL 2:US 2,535,861
PTL 3:JP 2008-174494 A

Summary of Invention

Technical Problem

However, as a result of studies by the present inventors, it has been found that the methods proposed in PTLs 1 and 2 can inhibit anionic polymerization of 1,1-dicyanoethylene, but are not sufficient to inhibit radical polymerization.

In addition, since 1,1-dicyanoethylene has higher reactivity with a Lewis basic compound than methylenemalonic acid, the contents taught by PTL 3 do not immediately apply to 1,1-dicyanoethylene.

Thus, there is still a need to enhance the storage stability of 1,1-dicyanoethylene-containing compositions in order to prevent reaction and curing before use. In addition, when a 1,1-dicyanoethylene-containing composition having excellent storage stability can be provided, it is expected that a cured product and a laminate that do not impair the expected physical properties can be provided.

On the other hand, phenolic compounds are generally used as radical polymerization inhibitors. However, in a system in which 1,1-dicyanoethylene is present, 1,1-dicyanoethylene may react with a hydroxy group of the phenolic compound, and polymerization of 1,1-dicyanoethylene may not be sufficiently suppressed.

An object of the present invention is to provide a 1,1-dicyanoethylene-containing composition having excellent storage stability. Another object of the present invention is to provide a cured product using the same components as those of the 1,1-dicyanoethylene-containing composition, and a laminate containing the cured product.

Solution to Problem

The present inventors conducted extensive studies and found that a 1,1-dicyanoethylene-containing composition having excellent storage stability can be provided by stabilizing a hydroxy group of a phenolic compound in a system in which the phenolic compound is present in 1,1-dicyanoethylene. Specifically, as a method for stabilizing the hydroxy group of the phenolic compound, a method of introducing a specific substituent into the phenolic compound and a method of allowing a specific compound that stabilizes the hydroxy group of the phenolic compound to coexist have been found.

Specifically, the present invention provides the following [1] to [40].

[1] A 1,1-dicyanoethylene-containing composition containing 1,1-dicyanoethylene and an o-substituted phenol compound (A-1) represented by the following formula (I):

[Chem. 1]

(I)

in which $R^{11}$ and $R^{15}$ are each independently a hydrogen atom or an o-substituent which is one selected from the group consisting of an alkyl group, a cycloalkyl group, an aryl group, an alkoxy group, a carboxy group, an ester group represented by -COOR$^{1a}$ (where $R^{1a}$ is an alkyl group having 1 to 25 carbon atoms), an acid anhydride group, an acyl group represented by -COR$^{1b}$ (where $R^{1b}$ is an alkyl group having 1 to 25 carbon atoms), an acyloxy group represented by -OCOR$^{1c}$ (where $R^{1c}$ is an alkyl group having 1 to 25 carbon atoms), a halogen atom, and a haloalkyl group (provided that a case in which both of $R^{11}$ and $R^{15}$ are hydrogen atoms is excluded);
$R^{13}$ is a hydrogen atom or a p-substituent, and the p-substituent is the same as the o-substituent; and
$R^{12}$ and $R^{14}$ are each independently a hydrogen atom or a m-substituent, and the m-substituent is the same as the p-substituent.

[2] The 1,1-dicyanoethylene-containing composition according to [1], in which one or both of $R^{11}$ and $R^{15}$ have a Taft steric parameter Es value of less than 1.24.
[3] The 1,1-dicyanoethylene-containing composition according to [1] or [2], in which one or both of $R^{11}$ and $R^{15}$ are each independently one selected from the group consisting of a branched alkyl group, a cycloalkyl group, and an aryl group.
[4] The 1,1-dicyanoethylene-containing composition according to any one of [1] to [3], in which the o-substituted phenol compound (A-1) is at least one selected from 2,6-di-tert-butylphenol, 2,6-di-tert-butyl-4-methylphenol, pentaerythritol tetrakis[3-(3,5-di-tert-butyl-4-hydroxyphenyl)propionate], octadecyl 3-(3,5-di-tert-butyl-4-hydroxyphenyl)propionate, 2,4-di-tert-butylphenol, 1,1,3-tris(2-methyl-4-hydroxy-5-tert-butylphenyl)butane, and 4,4'-butylidenebis(6-tert-butyl-m-cresol).
[5] The 1,1-dicyanoethylene-containing composition according to [1], in which both of $R^{11}$ and $R^{15}$ have a Taft steric parameter Es value of less than 1.24.
[6] The 1,1-dicyanoethylene-containing composition according to any one of [1] to [5], in which both of $R^{11}$ and $R^{15}$ are each independently one selected from the group consisting of a branched alkyl group, a cycloalkyl group, and an aryl group.
[7] The 1,1-dicyanoethylene-containing composition according to any one of [1] to [3], in which the o-substituted phenol compound (A-1) contains at least one selected from 2,6-di-tert-butylphenol, 2,6-di-tert-butyl-4-methylphenol, pentaerythritol tetrakis[3-(3,5-di-tert-butyl-4-hydroxyphenyl)propionate], and octadecyl 3-(3,5-di-tert-butyl-4-hydroxyphenyl)propionate.
[8] The 1,1-dicyanoethylene-containing composition according to any one of [1] to [7], excluding a case where both $R^{11}$ and $R^{15}$ are halogen atoms.
[9] The 1,1-dicyanoethylene-containing composition according to any one of [1] to [8], further containing a polymerizable monomer (B).
[10] The 1,1-dicyanoethylene-containing composition according to [9], in which the polymerizable monomer (B) is one or more selected from the group consisting of ethylene, propylene, butadiene, isobutylene, isoprene, 1-hexene, 1-octene, vinyl acetate, vinyl propionate, vinyl butyrate, styrene, $\alpha$-methylstyrene, p-methylstyrene, acrylic acid, methacrylic acid, alkyl acrylate, alkyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester.
[11] The 1,1-dicyanoethylene-containing composition according to [10], in which the polymerizable monomer (B) contains 2-cyanoacrylic acid alkyl ester.
[12] The 1,1-dicyanoethylene-containing composition according to [11], in which the 2-cyanoacrylic acid alkyl ester is ethyl 2-cyanoacrylate.
[13] A cured product obtained by reacting 1,1-dicyanoethylene with a Lewis basic compound in the presence of an o-substituted phenol compound (A-1) represented by the following formula (I):

[Chem. 2]

$$\text{(I)}$$

in which $R^{11}$ and $R^{15}$ are each independently a hydrogen atom or an o-substituent which is one selected from the group consisting of an alkyl group, a cycloalkyl group, an aryl group, an alkoxy group, a carboxy group, an ester group represented by -$COOR^{1a}$ (where $R^{1a}$ is an alkyl group having 1 to 25 carbon atoms), an acid anhydride group, an acyl group represented by -$COR^{1b}$ (where $R^{1b}$ is an alkyl group having 1 to 25 carbon atoms), an acyloxy group represented by -$OCOR^{1c}$ (where $R^{1c}$ is an alkyl group having 1 to 25 carbon atoms), a halogen atom, and a haloalkyl group (provided that a case in which both of $R^{11}$ and $R^{15}$ are hydrogen atoms is excluded);
$R^{13}$ is a hydrogen atom or a p-substituent, and the p-substituent is the same as the o-substituent; and
$R^{12}$ and $R^{14}$ are each independently a hydrogen atom or a m-substituent, and the m-substituent is the same as the p-substituent.

[14] The cured product according to [13], in which one or both of $R^{11}$ and $R^{15}$ have a Taft steric parameter Es value of less than 1.24.
[15] The cured product according to [13] or [14], in which one or both of $R^{11}$ and $R^{15}$ are each independently one selected from the group consisting of a branched alkyl group, a cycloalkyl group, and an aryl group.
[16] The cured product according to any one of [13] to [15], in which the o-substituted phenol compound (A-1) is at least one selected from 2,6-di-tert-butylphenol, 2,6-di-tert-butyl-4-methylphenol, pentaerythritol tetrakis[3-(3,5-di-tert-butyl-4-hydroxyphenyl)propionate], octadecyl 3-(3,5-di-tert-butyl-4-hydroxyphenyl)propionate, 2,4-di-tert-butylphenol, 1,1,3-tris(2-methyl-4-hydroxy-5-tert-butylphenyl)butane, and 4,4'-butylidenebis(6-tert-butyl-m-cresol).
[17] The cured product according to [13], in which both of $R^{11}$ and $R^{15}$ have a Taft steric parameter Es value of less than 1.24.
[18] The cured product according to any one of [13] to [17], in which both of $R^{11}$ and $R^{15}$ are each independently one selected from the group consisting of a branched alkyl group, a cycloalkyl group, and an aryl group.
[19] The cured product according to any one of [13] to [15], in which the o-substituted phenol compound (A-1) contains at least one selected from 2,6-di-tert-butylphenol, 2,6-di-tert-butyl-4-methylphenol, pentaerythritol tetrakis[3-(3,5-di-tert-butyl-4-hydroxyphenyl)propionate], and octadecyl 3-(3,5-di-tert-butyl-4-hydroxyphenyl)propionate.
[20] The cured product according to any one of [13] to [19], excluding a case where both $R^{11}$ and $R^{15}$ are halogen atoms.
[21] The cured product according to [13], which is obtained by reacting 1,1-dicyanoethylene, a polymerizable monomer (B), and the Lewis basic compound.
[22] The cured product according to [21], in which the polymerizable monomer (B) is one or more selected from the group consisting of ethylene, propylene, butadiene, isobutylene, isoprene, 1-hexene, 1-octene, vinyl acetate, vinyl propionate, vinyl butyrate, styrene, $\alpha$-methylstyrene, p-methylstyrene, acrylic acid, methacrylic acid, alkyl acrylate, alkyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester.
[23] The cured product according to [22], in which the polymerizable monomer (B) contains 2-cyanoacrylic acid alkyl ester.
[24] The cured product according to [23], in which the 2-cyanoacrylic acid alkyl ester is ethyl 2-cyanoacrylate.
[25] A laminate containing the cured product according to any one of [13] to [24] and an adherend bonded to the cured product.
[26] A 1,1-dicyanoethylene-containing composition containing 1,1-dicyanoethylene, a phenol compound (A-2) represented by the following formula (II), and a compound (C) having a proton affinity (Epa) of 780 kJ/mol or less and a pKa of 5 or less in the form of an aqueous solution:

[Chem. 3]

(II)

in which $R^{21}$ and $R^{25}$ are each independently a hydrogen atom or an o-substituent which is one selected from the group consisting of an alkyl group, a cycloalkyl group, an aryl group, an alkoxy group, a carboxy group, an ester group represented by $-COOR^{2a}$ (where $R^{2a}$ is an alkyl group having 1 to 25 carbon atoms), an acid anhydride group, an acyl group represented by $-COR^{2b}$ (where $R^{2b}$ is a hydrogen atom or an alkyl group having 1 to 25 carbon atoms), an acyloxy group represented by $-OCOR^{2c}$ (where $R^{2c}$ is an alkyl group having 1 to 25 carbon atoms), a hydroxy group, an amino group, a halogen atom, and a haloalkyl group;
$R^{23}$ is a hydrogen atom or a p-substituent, and the p-substituent is the same as the o-substituent; and
$R^{22}$ and $R^{24}$ are each independently a hydrogen atom or a m-substituent, and the m-substituent is the same as the p-substituent.

[27] The 1,1-dicyanoethylene-containing composition according to [26], in which both $R^{21}$ and $R^{25}$ are hydrogen atoms.
[28] The 1,1-dicyanoethylene-containing composition according to [26] or [27], in which the compound (C) is at least one selected from the group consisting of methanesulfonic acid, sulfuric acid, and sulfur dioxide.
[29] The 1,1-dicyanoethylene-containing composition according to any one of [26] to [28], further containing a polymerizable monomer (B).
[30] The 1,1-dicyanoethylene-containing composition according to [29], in which the polymerizable monomer (B) is one or more selected from the group consisting of ethylene, propylene, butadiene, isobutylene, isoprene, 1-hexene, 1-octene, vinyl acetate, vinyl propionate, vinyl butyrate, styrene, $\alpha$-methylstyrene, p-methylstyrene, acrylic acid, methacrylic acid, alkyl acrylate, alkyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester.
[31] The 1,1-dicyanoethylene-containing composition according to [30], in which the polymerizable monomer (B) contains 2-cyanoacrylic acid alkyl ester.
[32] The 1,1-dicyanoethylene-containing composition according to [31], in which the 2-cyanoacrylic acid alkyl ester is ethyl 2-cyanoacrylate.
[33] A cured product obtained by reacting 1,1-dicyanoethylene with a Lewis basic compound in the presence of a phenol compound (A-2) represented by the following formula (II) and a compound (C) having a proton affinity (Epa) of 780 kJ/mol or less and a pKa of 5 or less in the form of an aqueous solution:

[Chem. 4]

(II)

in which $R^{21}$ and $R^{25}$ are each independently a hydrogen atom or an o-substituent which is one selected from the group consisting of an alkyl group, a cycloalkyl group, an aryl group, an alkoxy group, a carboxy group, an ester group represented by $-COOR^{2a}$ (where $R^{2a}$ is an alkyl group having 1 to 25 carbon atoms), an acid anhydride

group, an acyl group represented by $-COR^{2b}$ (where $R^{2b}$ is a hydrogen atom or an alkyl group having 1 to 25 carbon atoms), an acyloxy group represented by $-OCOR^{2c}$ (where $R^{2c}$ is an alkyl group having 1 to 25 carbon atoms), a hydroxy group, an amino group, a halogen atom, and a haloalkyl group;
$R^{23}$ is each independently a hydrogen atom or a p-substituent, and the p-substituent is the same as the o-substituent; and
$R^{22}$ and $R^{24}$ are each independently a hydrogen atom or a m-substituent, and the m-substituent is the same as the p-substituent.

[34] The cured product according to [33], in which both $R^{21}$ and $R^{25}$ are hydrogen atoms.
[35] The cured product according to [33] or [34], in which the compound (C) is at least one selected from the group consisting of methanesulfonic acid, sulfuric acid, and sulfur dioxide.
[36] The cured product according to any one of [33] to [35], which is obtained by reacting 1,1-dicyanoethylene, a polymerizable monomer (B), and the Lewis basic compound.
[37] The cured product according to [36], in which the polymerizable monomer (B) is one or more selected from the group consisting of ethylene, propylene, butadiene, isobutylene, isoprene, 1-hexene, 1-octene, vinyl acetate, vinyl propionate, vinyl butyrate, styrene, $\alpha$-methylstyrene, p-methylstyrene, acrylic acid, methacrylic acid, alkyl acrylate, alkyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester.
[38] The cured product according to [37], in which the polymerizable monomer (B) contains 2-cyanoacrylic acid alkyl ester.
[39] The cured product according to [38], in which the 2-cyanoacrylic acid alkyl ester is ethyl 2-cyanoacrylate.
[40] A laminate containing the cured product according to any one of [33] to [39] and an adherend bonded to the cured product.

Advantageous Effects of Invention

According to the present invention, it is possible to provide a 1,1-dicyanoethylene-containing composition having excellent storage stability. In addition, according to the present invention, it is possible to provide a cured product using the same components as those of the 1,1-dicyanoethylene-containing composition, and a laminate containing the cured product.

Description of Embodiments

Hereinafter, a description will be given based on an example of a mode for carrying out the present invention (hereinafter, sometimes referred to as "the present embodiment"). However, the embodiments described below are illustrative for embodying the technological thought of the present invention, and the present invention is not limited to the following descriptions.
In addition, in the description herein, preferred modes of the embodiments are shown, but a combination of two or more of individual preferred modes is also a preferred mode. Regarding the matters indicated by numerical ranges, in a case where there are several numerical ranges, it is possible to selectively combine a lower limit value and an upper limit value thereof to obtain a preferred mode.
In the description herein, when there is a description pertaining to a numerical range of "XX to YY", the description means "XX or more and YY or less".

[First Embodiment]

1, 1-Dicyanoethylene-Containing Composition According to First Embodiment]

A 1,1-dicyanoethylene-containing composition according to a first embodiment of the present invention is a 1,1-dicyanoethylene-containing composition containing 1,1-dicyanoethylene and a specific o-substituted phenol compound (A-1). When the 1,1-dicyanoethylene-containing composition contains a specific o-substituted phenol compound (A-1), a 1,1-dicyanoethylene-containing composition having excellent storage stability can be provided. The specific o-substituted phenol compound (A-1) may be one kind or a plurality of kinds.

(1,1-Dicyanoethylene)

1,1-Dicyanoethylene may be produced according to the production method described in J. Am. Chem. Soc., 1989, 111, 9078-9081 or the production method described in U.S. Patent No. 2476270. The purity of 1,1-dicyanoethylene

to be used in the 1,1-dicyanoethylene-containing composition according to the embodiment of the present invention is preferably 95% or more, more preferably 97% or more, still more preferably 98% or more, and yet still more preferably 99% or more. The purity of 1,1-dicyanoethylene can be determined by, for example, gas chromatography.

In addition, the produced 1,1-dicyanoethylene may be stored in the state of coexisting with, for example, an aromatic solvent such as toluene or xylene; an aliphatic solvent such as hexane or heptane; a naphthene solvent such as cyclohexane; an ester solvent such as ethyl acetate; or an ether solvent such as tetrahydrofuran or diethyl ether until immediately before use.

(Specific o-Substituted Phenol Compound (A-1))

The specific o-substituted phenol compound (A-1) is represented by the following formula (I).

[Chem. 5]

OH
$R^{15}$ $R^{11}$
$R^{14}$ $R^{12}$
$R^{13}$

(I)

In the formula (I), $R^{11}$ and $R^{15}$ are each independently a hydrogen atom or an o-substituent. The o-substituent refers to a substituent located at the o-position relative to a hydroxy group (the -OH group in the formula (I)) of the phenol compound. However, in the first embodiment, a case where both $R^{11}$ and $R^{15}$ (that is, both of the two o-positions) are hydrogen atoms is excluded. When both $R^{11}$ and $R^{15}$ (that is, both of the two o-positions) are not hydrogen atoms, the o-substituent can stabilize the hydroxy group of the phenol compound. The reason why the hydroxy group is stabilized is not limited thereto, but it is considered that the o-substituent in the vicinity of the hydroxy group becomes a steric hindrance and suppresses the reactivity of the hydroxy group. Then, it is considered that by stabilizing the hydroxy group of the phenol compound, the reaction with the coexisting 1,1-dicyanoethylene can be suppressed. The reaction between 1,1-dicyanoethylene and the phenol compound can be confirmed by thickening or solidification (precipitation) of the composition, as exemplified in the section of Examples.

In the formula (I), $R^{11}$ and $R^{15}$ are each independently a hydrogen atom or an o-substituent which is one selected from the group consisting of an alkyl group, a cycloalkyl group, an aryl group, an alkoxy group, a carboxy group, an ester group represented by $-COOR^{1a}$ (where $R^{1a}$ is an alkyl group having 1 to 25 carbon atoms), an acid anhydride group, an acyl group represented by $-COR^{1b}$ (where $R^{1b}$ is an alkyl group having 1 to 25 carbon atoms), an acyloxy group represented by $-OCOR^{1c}$ (where $R^{1c}$ is an alkyl group having 1 to 25 carbon atoms), a halogen atom, and a haloalkyl group.

That is, in the formula (I), $R^{11}$ and $R^{15}$ are each independently a hydrogen atom, an alkyl group, a cycloalkyl group, an aryl group, an alkoxy group, a carboxy group, an ester group represented by $-COOR^{1a}$ (where $R^{1a}$ is an alkyl group having 1 to 25 carbon atoms), an acid anhydride group, an acyl group represented by $-COR^{1b}$ (where $R^{1b}$ is an alkyl group having 1 to 25 carbon atoms), an acyloxy group represented by $-OCOR^{1c}$ (where $R^{1c}$ is an alkyl group having 1 to 25 carbon atoms), a halogen atom, or a haloalkyl group.

The alkyl group that may be represented by $R^{11}$ and $R^{15}$ is preferably an alkyl group having 1 to 25 carbon atoms, and examples thereof include a methyl group, an ethyl group, a n-propyl group, an isopropyl group, a n-butyl group, an isobutyl group, a sec-butyl group, a tert-butyl group, a n-pentyl group, an isopentyl group, a neopentyl group, and a n-hexyl group.

The cycloalkyl group that may be represented by $R^{11}$ and $R^{15}$ is preferably a cycloalkyl group having 3 to 12 carbon atoms, and examples thereof include a cyclopropyl group, a cyclobutyl group, a cyclopentyl group, and a cyclohexyl group.

The aryl group that may be represented by $R^{11}$ and $R^{15}$ is preferably an aryl group having 6 to 25 carbon atoms, and examples thereof include a phenyl group, a tolyl group, a xylyl group, and a naphthyl group.

The alkoxy group that may be represented by $R^{11}$ and $R^{15}$ is preferably an alkoxy group having 1 to 25 carbon atoms, and examples thereof include a methoxy group, an ethoxy group, a n-propoxy group, an isopropoxy group, a n-butoxy group, an isobutoxy group, a sec-butoxy group, and a tert-butoxy group.

In the ester group ($-COOR^{1a}$), the acyl group ($-COR^{1b}$), and the acyloxy group ($-OCOR^{1c}$) that may be

represented by $R^{11}$ and $R^{15}$, examples of $R^{1a}$, $R^{1b}$ and $R^{1c}$ include a methyl group, an ethyl group, a n-propyl group, an isopropyl group, a n-butyl group, an isobutyl group, a sec-butyl group, a tert-butyl group, a n-pentyl group, an isopentyl group, a neopentyl group, a n-hexyl group, a heptyl group, an octyl group, a decyl group, a dodecyl group, and an octadecyl group.

Examples of the acid anhydride group that may be represented by $R^{11}$ and $R^{15}$ include acid anhydride groups derived from phthalic anhydride, maleic anhydride, trimellitic anhydride, pyromellitic anhydride, hexahydrophthalic anhydride, tetrahydrophthalic anhydride, methylnadic anhydride, nadic anhydride, glutaric anhydride, dimethylglutaric anhydride, diethylglutaric anhydride, succinic anhydride, methylhexahydrophthalic anhydride, and methyltetrahydrophthalic anhydride.

Examples of the halogen atom that may be represented by $R^{11}$ and $R^{15}$ include a fluorine atom, a chlorine atom, a bromine atom, and an iodine atom. The haloalkyl group that may be represented by $R^{11}$ and $R^{15}$ is preferably a haloalkyl group having 1 to 12 carbon atoms, and more preferably a haloalkyl group having 1 to 6 carbon atoms. Examples of the halogen atom constituting the haloalkyl group include a fluorine atom, a chlorine atom, a bromine atom, and an iodine atom.

Specific examples of the o-substituent that may be represented by $R^{11}$ and $R^{15}$ include a linear alkyl group having 1 to 25 carbon atoms, preferably 1 to 12 carbon atoms, and more preferably 1 to 6 carbon atoms, a branched alkyl group having 3 to 25 carbon atoms, preferably 3 to 12 carbon atoms, and more preferably 3 to 6 carbon atoms, a cycloalkyl group having 5 to 25 carbon atoms, preferably 5 to 12 carbon atoms, and more preferably 5 to 6 carbon atoms, an alkoxy group having 1 to 25 carbon atoms, preferably 1 to 12 carbon atoms, and more preferably 1 to 6 carbon atoms, and an aryl group having 6 to 25 carbon atoms, preferably 6 to 12 carbon atoms, and more preferably 6 carbon atoms. Among these, the o-substituent is preferably a linear alkyl group having 1 to 6 carbon atoms, a branched alkyl group having 3 to 6 carbon atoms, a cycloalkyl group having 5 to 12 carbon atoms, or an aryl group having 5 to 12 carbon atoms, more preferably a methyl group, an ethyl group, a n-propyl group, an isopropyl group, a n-butyl group, a sec-butyl group, a tert-butyl group, a n-pentyl group, a cyclohexyl group, or a phenyl group, still more preferably a tert-butyl group, a cyclohexyl group, or a phenyl group, and yet still more preferably a tert-butyl group.

In a preferable embodiment, one or both of $R^{11}$ and $R^{15}$ have a Taft steric parameter Es value (hereinafter referred to as "Taft value") of less than 1.24. As the Taft value, a value described in NPL 1 (John A. M., ANNICK P. and JACQUSE-EMI LE D. Tetrahedron, 1978, 34, 3553-3562) can be usually adopted.

The Taft value is preferably less than 0.08, more preferably 0.01 or less, still more preferably -0.06 or less, yet still more preferably less than -0.16, yet still more preferably -0.78 or less, and yet still more preferably -1.00 or less. Examples of the substituent having a Taft value of -1.00 or less include a sec-butyl group and a tert-butyl group.

Preferably, both of $R^{11}$ and $R^{15}$ have a Taft steric parameter Es value (Taft value) of less than 1.24. A more preferable range of the Taft value is as described above.

In another preferable embodiment, one or both of $R^{11}$ and $R^{15}$ are each independently one selected from the group consisting of a branched alkyl group, a cycloalkyl group, and an aryl group. Preferably, one or both of $R^{11}$ and $R^{15}$ are each independently one selected from the group consisting of a branched alkyl group, a cycloalkyl group, and an aryl group. Examples of the branched alkyl group, the cycloalkyl group, and the aryl group include those described above.

In addition, in one embodiment, a case where both $R^{11}$ and $R^{15}$ are halogen atoms is excluded. As a result, the expected effect can be more reliably exhibited.

In the formula (I), $R^{13}$ is a hydrogen atom or a p-substituent, and the p-substituent is the same as the o-substituent. When the p-substituent is the same as the o-substituent, it means that examples of the p-substituent are the same as those of the o-substituent. Therefore, the p-substituent and the o-substituent may be the same or different from each other. The p-substituent refers to a substituent located at the p-position relative to a hydroxy group (the -OH group in the formula (I)) of the phenol compound. That is, in the formula (I), $R^{13}$ is a hydrogen atom, an alkyl group, a cycloalkyl group, an aryl group, an alkoxy group, a carboxy group, an ester group represented by $-COOR^{1a}$ (where $R^{1a}$ is an alkyl group having 1 to 25 carbon atoms), an acid anhydride group, an acyl group represented by $-COR^{1b}$ (where $R^{1b}$ is an alkyl group having 1 to 25 carbon atoms), an acyloxy group represented by $-OCOR^{1c}$ (where $R^{1c}$ is an alkyl group having 1 to 25 carbon atoms), a halogen atom, or a haloalkyl group.

Examples of the p-substituent that may be represented by $R^{13}$ include: a linear alkyl group having 1 to 25 carbon atoms, preferably 1 to 12 carbon atoms, and more preferably 1 to 6 carbon atoms; a branched alkyl group having 3 to 25 carbon atoms, preferably 3 to 12 carbon atoms, and more preferably 3 to 6 carbon atoms; a cycloalkyl group having 5 to 25 carbon atoms, preferably 5 to 12 carbon atoms, and more preferably 5 to 6 carbon atoms; an alkoxy group having 1 to 25 carbon atoms, preferably 1 to 12 carbon atoms, and more preferably 1 to 6 carbon atoms; an aryl group having 6 to 25 carbon atoms, preferably 6 to 12 carbon atoms, and more preferably 6 carbon atoms; a combination of an alkyl group having 1 to 25 carbon atoms, preferably 1 to 12 carbon atoms, and more preferably 1 to 6 carbon atoms and $-COOR^{1a}$ (where $R^{1a}$ is an alkyl group having 1 to 25 carbon atoms); and a combination of an alkyl group having 1 to 25 carbon atoms, preferably 1 to 12 carbon atoms, and more preferably 1 to 6 carbon atoms, $-COOR^{1a}$ (where $R^{1a}$ is an alkyl group having 1 to 25 carbon atoms), an alkyl group having 1 to 25 carbon atoms, preferably 1 to 12 carbon atoms, and more preferably 1 to

6 carbon atoms, -OCOR$^{1c}$ (where R$^{1c}$ is an alkyl group having 1 to 25 carbon atoms), an alkyl group having 1 to 25 carbon atoms, preferably 1 to 12 carbon atoms, and more preferably 1 to 6 carbon atoms, and an aryl group having 6 to 25 carbon atoms, preferably 6 to 12 carbon atoms, and more preferably 6 carbon atoms (this aryl group may have any substituent, for example, a hydroxy group or a tert-butyl group).

In the formula (I), R$^{12}$ and R$^{14}$ are each independently a hydrogen atom or a m-substituent, and the m-substituent is the same as the p-substituent. When the m-substituent is the same as the p-substituent, it means that examples of the m-substituent are the same as those of the p-substituent. Therefore, the m-substituent and the p-substituent may be the same or different from each other. The m-substituent refers to a substituent located at the m-position relative to a hydroxy group (the -OH group in the formula (I)) of the phenol compound. That is, in the formula (I), R$^{12}$ and R$^{14}$ are each independently a hydrogen atom, an alkyl group, a cycloalkyl group, an aryl group, an alkoxy group, a carboxy group, an ester group represented by -COOR$^{1a}$ (where R$^{1a}$ is an alkyl group having 1 to 25 carbon atoms), an acid anhydride group, an acyl group represented by -COR$^{1b}$ (where R$^{1b}$ is an alkyl group having 1 to 25 carbon atoms), an acyloxy group represented by -OCOR$^{1c}$ (where R$^{1c}$ is an alkyl group having 1 to 25 carbon atoms), a halogen atom, or a haloalkyl group.

R$^{12}$ and R$^{14}$ are each independently preferably a hydrogen atom, or one selected from the group consisting of a linear alkyl group having 1 to 25 carbon atoms, preferably 1 to 12 carbon atoms, and more preferably 1 to 6 carbon atoms, and a branched alkyl group having 3 to 25 carbon atoms, preferably 3 to 12 carbon atoms, and more preferably 3 to 6 carbon atoms, and more preferably a hydrogen atom.

Specific examples of the specific o-substituted phenol compound (A-1) include 2,6-di-tert-butyl-4-methylphenol, 2,6-di-tert-butylphenol, pentaerythritol tetrakis[3-(3,5-di-tert-butyl-4-hydroxyphenyl)propionate], octadecyl 3-(3,5-di-tert-butyl-4-hydroxyphenyl)propionate, 2,4-di-tert-butylphenol, 2,2'-methylenebis(4-methyl-6-tert-butylphenol), 2,2'-methylenebis-(4-ethyl-6-tert-butylphenol) (YOSHINOX 425), 4,4'-butylidenebis-(6-tert-butyl-3-methylphenol) (YOSHINOX BB), 2,2'-methylenebis[6-tert-butyl-4-methylphenol] (SUMILIZER MDP-S), 1,1,3-tris(2-methyl-4-hydroxy-5-tert-butylphenyl)butane (ADK STAB AO-30), 4,4'-butylidenebis(6-tert-butyl-m-cresol) (ADK STAB AO-40), octadecyl 3-(3,5-di-tert-butyl-4-hydroxyphenyl)propionate (ADK STAB AO-50), 2,2'-dimethyl-2,2'-(2,4,8,10-tetraoxaspiro[5.5]undecane-3,9-diyl)dipropane-1,1'-diyl=bis[3-(3-tert-butyl-4-hydroxy-5-methylphenyl)propanoate] (ADK STAB AO-80), 1,3,5-trimethyl-2,4,6-tris(3,5-di-tert-butyl-4-hydroxybenzyl)benzene (ADK STAB AO-330), 4,4'-thiobis[3-methyl-6-(tert-butyl)phenol] (SUMILIZER WX-R), 2,6-bis[(2-hydroxy-5-methylphenyl)methyl]-4-methylphenol, 2,4,6-tris(3', 5'-di-tert-butyl-4'-hydroxybenzyl)mesitylene, 1'-hydroxy[2,2'-ethylidenebis[4,6-bis(1,1-dimethylpropyl)benzene]]-1-yl acrylate (SUMILIZER GS), 2-tert-butyl-4-methyl-6-(2-hydroxy-3-tert-butyl-5-methylbenzyl)phenyl acrylate (SUMILIZER GM), and 2-tert-butyl-6-methyl-4-{3-[(2,4,8,10-tetra-tert-butyldibenzo[d,f][1,3,2]dioxaphosphepin-6-yl)oxy] propyl}phenol (SUMILIZER GP). Of these, compounds belonging to o-disubstituted phenols are preferred. In a preferable embodiment, the specific o-substituted phenol compound (A-1) is at least one selected from 2,6-di-tert-butylphenol, 2,6-di-tert-butyl-4-methylphenol, pentaerythritol tetrakis[3-(3,5-di-tert-butyl-4-hydroxyphenyl)propionate], octadecyl 3-(3,5-di-tert-butyl-4-hydroxyphenyl)propionate, 2,4-di-tert-butylphenol, 1,1,3-tris(2-methyl-4-hydroxy-5-tert-butylphenyl)butane, and 4,4'-butylidenebis(6-tert-butyl-m-cresol). In a more preferable embodiment, the specific o-substituted phenol compound (A-1) is at least one selected from 2,6-di-tert-butylphenol, 2,6-di-tert-butyl-4-methylphenol, pentaerythritol tetrakis[3-(3,5-di-tert-butyl-4-hydroxyphenyl)propionate], octadecyl 3-(3,5-di-tert-butyl-4-hydroxyphenyl)propionate, and 2,4-di-tert-butylphenol. In a still more preferable embodiment, the specific o-substituted phenol compound (A-1) is at least one selected from 2,6-di-tert-butylphenol, 2,6-di-tert-butyl-4-methylphenol, pentaerythritol tetrakis[3-(3,5-di-tert-butyl-4-hydroxyphenyl) propionate], and octadecyl 3-(3,5-di-tert-butyl-4-hydroxyphenyl)propionate. In a yet still more preferable embodiment, the specific o-substituted phenol compound (A-1) contains at least one selected from 2,6-di-tert-butylphenol and 2,6-di-tert-butyl-4-methylphenol. In a case where a compound belonging to the o-monosubstituted phenol is used, it is preferable that an acidic compound other than the phenolic compound is used in combination, and it is more preferable that the compound (C) described later in the second embodiment is used in combination.

From the viewpoint of suppressing coloration of the 1,1-dicyanoethylene-containing composition, the specific o-substituted phenol compound (A-1) preferably has, as a p-substituent, a substituent other than a hydrogen atom, and preferably has a substituent other than a hydrogen atom and a halogen atom.

(Component (B): Polymerizable Monomer)

The 1,1-dicyanoethylene-containing composition according to the first embodiment may further contain a polymerizable monomer (B). The polymerizable monomer (B) excludes 1,1-dicyanoethylene. The polymerizable monomer (B) is preferably reactive with 1,1-dicyanoethylene. The polymerizable monomer (B) may be one kind or a plurality of kinds. The polymerizable monomer (B) may be a radically polymerizable monomer or an anionically polymerizable monomer.

Examples of the polymerizable monomer (B) include ethylene, propylene, butadiene, isobutylene, isoprene, 1-hexene, 1-octene, vinyl acetate, vinyl propionate, vinyl butyrate, styrene, $\alpha$-methylstyrene, p-methylstyrene, acrylic acid, methacrylic acid, alkyl acrylate esters such as butyl acrylate, alkyl methacrylate esters such as methyl methacrylate and

dodecyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester. Among these, from the viewpoint of excellent reactivity with 1,1-dicyanoethylene, the polymerizable monomer (B) is preferably one or more selected from the group consisting of ethylene, propylene, butadiene, isobutylene, isoprene, 1-hexene, 1-octene, vinyl acetate, vinyl propionate, vinyl butyrate, styrene, $\alpha$-methylstyrene, p-methylstyrene, acrylic acid, methacrylic acid, alkyl acrylate, alkyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester, more preferably 2-cyanoacrylic acid alkyl ester, and still more preferably 2-cyanoacrylic acid alkyl ester is ethyl 2-cyanoacrylate.

The method for producing the polymerizable monomer (B) is not particularly limited, and the polymerizable monomer (B) can be produced by a known method alone or in combination. The polymerizable monomer (B) may be a commercially available product.

(Other Component)

The 1,1-dicyanoethylene-containing composition according to the first embodiment may contain one or more other components.

Examples of such other components include Bronsted acidic compounds other than phenolic compounds, acid anhydrides, Lewis acidic compounds, thickeners, dehydrating agents, radical polymerization inhibitors (excluding the above-described phenolic compounds), plasticizers, pigments, organic solvents, rubbers, organic fillers, and inorganic fillers. The other components can be used in such an amount that the expected effect of the present invention is not impaired. The acid anhydride may also function as a dehydrating agent. The rubber may also function as an organic filler. The inorganic filler may also function as a thickener.

Examples of the acidic compound other than the phenolic compound include methanesulfonic acid, sulfuric acid, sulfur dioxide, hydrochloric acid, nitric acid, sulfurous acid, p-toluenesulfonic acid, and phosphoric acid. Among these, a compound belonging to the compound (C) described later in the second embodiment is preferably used.

In the case of using an acidic compound other than the phenolic compound as the other component, the amount of the acidic compound is preferably 10 parts by mass or less, more preferably 5.0 parts by mass or less, still more preferably 2.0 parts by mass or less, and yet still more preferably 1.0 parts by mass or less when the total amount of the monomers (1,1-dicyanoethylene and the polymerizable monomer (B) used as required) is taken as 100 parts by mass. From the viewpoint of further enhancing the storage stability of the 1,1-dicyanoethylene-containing composition, the amount of the acidic compound is preferably 0.0005 parts by mass or more, more preferably 0.001 parts by mass or more, and still more preferably 0.005 parts by mass or more when the total amount of the monomers is taken as 100 parts by mass.

The content of the acidic compound is preferably 0.0005% by mass or more, more preferably 0.0009% by mass or more, and still more preferably 0.004% by mass or more, and preferably 10% by mass or less, more preferably 5.0% by mass or less, still more preferably 2.0% by mass or less, and yet still more preferably 1.0% by mass or less, when the entire 1,1-dicyanoethylene-containing composition is taken as 100% by mass, from the viewpoint of further enhancing the storage stability of the 1,1-dicyanoethylene-containing composition.

(Content of Each Component)

The total amount of the monomers (1,1-dicyanoethylene and the polymerizable monomer (B) used as required) is preferably 70% by mass or more, more preferably 80% by mass or more, and still more preferably 90% by mass or more, when the entire 1,1-dicyanoethylene-containing composition according to the first embodiment is taken as 100% by mass, from the viewpoint of achieving the expected physical properties. When the polymerizable monomer (B) is used, the mass ratio of 1,1-dicyanoethylene to the polymerizable monomer (B) is not particularly limited, and may be 99:1 to 1:99, 80:20 to 20:80, or 70:30 to 30:70.

The specific o-substituted phenol compound (A-1) is used in an amount of preferably 10 parts by mass or less, more preferably 5.0 parts by mass or less, still more preferably 2.0 parts by mass or less, and yet still more preferably 1.5 parts by mass or less, when the total amount of the monomers (1,1-dicyanoethylene and the polymerizable monomer (B) used as required) is taken as 100 parts by mass. From the viewpoint of enhancing the storage stability of the 1,1-dicyanoethylene-containing composition, the specific o-substituted phenol compound (A-1) is used in an amount of preferably 0.0005 parts by mass or more, more preferably 0.001 parts by mass or more, and still more preferably 0.005 parts by mass or more when the total amount of the monomers is taken as 100 parts by mass.

The content of the specific o-substituted phenol compound (A-1) is preferably 0.00003% by mass or more, more preferably 0.00006% by mass or more, and still more preferably 0.00009% by mass or more, and preferably 10% by mass or less, more preferably 5.0% by mass or less, still more preferably 2.0% by mass or less, and yet still more preferably 1.5% by mass or less, when the entire 1,1-dicyanoethylene-containing composition is taken as 100% by mass, from the

viewpoint of enhancing the storage stability of the 1,1-dicyanoethylene-containing composition.

(Application)

The 1,1-dicyanoethylene-containing composition according to the present embodiment can be used as an (instant) adhesive. Such adhesive applications include general household adhesive, medical applications, hemostatic adhesive, laminations, bookbinding, shoe assembly, automotive parts, air conditioners, electrical or electronic equipment components or other durable goods, assembly of components used in the building industry (e.g., insulating applications, thermo- and/or acoustical applications), packaging, die attach applications, wound closure, surgical sutures, medical device applications and labelling of all kinds, lash extension adhesive, and cosmetic adhesive.

An instant adhesive containing ethyl 2-cyanoacrylate as a main raw material is widely known, but ethyl 2-cyanoacrylate has a disadvantage of being inferior in heat resistance and moisture resistance, and therefore the instant adhesive is inferior in heat resistance and moisture resistance. On the other hand, 1,1-dicyanoethylene has excellent heat resistance and moisture resistance, and an adhesive using the 1,1-dicyanoethylene-containing composition according to the present embodiment has excellent heat resistance and moisture resistance.

The 1,1-dicyanoethylene-containing composition according to the present embodiment can also be used as a coating material. Examples of the application of such a coating material include a film capacitor, an insulating layer of an EL element, an electrostatic induction conversion element, a sensor (for example, a touch sensor, a vibration sensor, a biosensor, and a tire sensor (particularly, a sensor installed on a tire inner surface)), an actuator, a touch panel, a haptic device, a vibration power generation apparatus (for example, a vibration power generation floor and a vibration power generation tire), a speaker, a microphone, a vibration damping sheet, a hollow fiber membrane for water purification, and a resist film. An example of the haptic device is a device having a function of feeding back a tactile sense to a user.

[Cured Product According to First Embodiment]

The cured product according to the first embodiment of the present invention is a cured product obtained by reacting 1,1-dicyanoethylene with a Lewis basic compound in the presence of an o-substituted phenol compound (A-1) represented by the following formula (I).

[Chem. 6]

OH, $R^{11}$, $R^{12}$, $R^{13}$, $R^{14}$, $R^{15}$

(I)

in which $R^{11}$ and $R^{15}$ are each independently a hydrogen atom or an o-substituent which is one selected from the group consisting of an alkyl group, a cycloalkyl group, an aryl group, an alkoxy group, a carboxy group, an ester group represented by $-COOR^{1a}$ (where $R^{1a}$ is an alkyl group having 1 to 25 carbon atoms), an acid anhydride group, an acyl group represented by $-COR^{1b}$ (where $R^{1b}$ is an alkyl group having 1 to 25 carbon atoms), an acyloxy group represented by $-OCOR^{1c}$ (where $R^{1c}$ is an alkyl group having 1 to 25 carbon atoms), a halogen atom, and a haloalkyl group (provided that a case in which both of $R^{11}$ and $R^{15}$ are hydrogen atoms is excluded); that is, in the formula (I), $R^{11}$ and $R^{15}$ are each independently a hydrogen atom, an alkyl group, a cycloalkyl group, an aryl group, an alkoxy group, a carboxy group, an ester group represented by $-COOR^{1a}$ (where $R^{1a}$ is an alkyl group having 1 to 25 carbon atoms), an acid anhydride group, an acyl group represented by $-COR^{1b}$ (where $R^{1b}$ is an alkyl group having 1 to 25 carbon atoms), an acyloxy group represented by $-OCOR^{1c}$ (where $R^{1c}$ is an alkyl group having 1 to 25 carbon atoms), a halogen atom, or a haloalkyl group.

$R^{13}$ is a hydrogen atom or a p-substituent, and the p-substituent is the same as the o-substituent; and that is, $R^{13}$ is a hydrogen atom, an alkyl group, a cycloalkyl group, an aryl group, an alkoxy group, a carboxy group, an ester group represented by $-COOR^{1a}$ (where $R^{1a}$ is an alkyl group having 1 to 25 carbon atoms), an acid anhydride group, an acyl group represented by $-COR^{1b}$ (where $R^{1b}$ is an alkyl group having 1 to 25 carbon atoms), an acyloxy group represented by $-OCOR^{1c}$ (where $R^{1c}$ is an alkyl group having 1 to 25 carbon atoms), a halogen atom, or a haloalkyl

group.
$R^{12}$ and $R^{14}$ are each independently a hydrogen atom or a m-substituent, and the m-substituent is the same as the p-substituent. That is, $R^{12}$ and $R^{14}$ are each independently a hydrogen atom, an alkyl group, a cycloalkyl group, an aryl group, an alkoxy group, a carboxy group, an ester group represented by $-COOR^{1a}$ (where $R^{1a}$ is an alkyl group having 1 to 25 carbon atoms), an acid anhydride group, an acyl group represented by $-COR^{1b}$ (where $R^{1b}$ is an alkyl group having 1 to 25 carbon atoms), an acyloxy group represented by $-OCOR^{1c}$ (where $R^{1c}$ is an alkyl group having 1 to 25 carbon atoms), a halogen atom, or a haloalkyl group.

The cured product according to the first embodiment of the present invention may be a cured product obtained by reacting the 1,1-dicyanoethylene-containing composition described above.

(Monomer Mixture)

1,1-dicyanoethylene may or may not constitute a monomer mixture together with the polymerizable monomer (B). In the case of a monomer mixture, the 1,1-dicyanoethylene and the o-substituted phenol compound (A-1) represented by the formula (I) are respectively the same as the 1,1-dicyanoethylene and the o-substituted phenol compound (A-1) represented by the formula (I) contained in the 1,1-dicyanoethylene-containing composition according to the first embodiment. The polymerizable monomer (B) that can be contained in the monomer mixture is the same as the polymerizable monomer (B) that can be contained in the 1,1-dicyanoethylene-containing composition according to the first embodiment. The monomer mixture may contain one or more other components. Such other components are the same as the other components that can be contained in the 1,1-dicyanoethylene-containing composition in the first embodiment. In one embodiment, the cured product is a cured product obtained by reacting 1,1-dicyanoethylene, a polymerizable monomer (B), and a Lewis basic compound in the presence of an o-substituted phenol compound (A-1) represented by the formula (I).

(Lewis Basic Compound)

The Lewis basic compound usually functions as a polymerization catalyst for 1,1-dicyanoethylene or the monomer mixture. Examples of the Lewis basic compound include water, alcohols, and alkylamines.
Examples of the alcohol include methanol, ethanol, and propanol.
Examples of the alkylamine include tertiary amines such as trimethylamine, triethylamine, tripropylamine, triisopropylamine, N,N-dimethylethylamine, N,N-dimethylpropylamine, and N,N-dimethylisopropylamine.
Among these, in one embodiment, water is used as the Lewis basic compound.

(Other Additives)

The cured product according to the present embodiment may contain, as other additives, at least one selected from the group consisting of thickeners (e.g., organic thickeners, inorganic thickeners such as silica), dehydrating agents (e.g., carboxylic acid anhydrides such as acetic anhydride, cyclic sulfonic acid esters such as propane sultone, and phosphoric acid anhydrides such as diphosphorus pentoxide), radical polymerization inhibitors (e.g., stable radical compounds, and metal salts), plasticizers (e.g., ester compounds such as phthalic acid esters and adipic acid esters), rubbers (e.g., natural rubbers, styrene-butadiene rubbers, hydrogenated styrene-butadiene rubbers, acrylic rubbers, nitrile rubbers, and hydrogenated nitrile rubbers), pigments, and fillers (e.g., inorganic fillers and organic fillers) in an amount that does not excessively impair the expected effect of the present invention. Specific examples of the organic thickener include polymer compounds such as an ethylene-vinyl acetate copolymer, a methyl (meth)acrylate resin, a polystyrene resin, a (modified) cellulose resin, and an acrylonitrile resin. Specific examples of the stable radical compound include TEMPO (2,2,6,6-tetramethylpiperidine 1-oxyl). The inorganic thickener may also function as an inorganic filler. The above-mentioned phenolic compound is excluded from the radical polymerization inhibitor. The rubber may also function as an organic filler. Further, a hardening accelerator such as a polyethylene glycol derivative, crown ether, and calixarene can be added for the purpose of improving the bonding speed. Furthermore, a filler, an elastomer, a thixotropy imparting agent, an adhesion imparting agent, a crosslinking agent, a fragrance, and the like may be added according to the purpose.

(Production Method of Cured Product)

The production method of the cured product is not particularly limited. For example, a cured product can be obtained by mixing the monomer mixture with the Lewis basic compound at room temperature (23°C).
The amount of the Lewis basic compound is not particularly limited, and is preferably 0.001 to 1.0 parts by mass,

and more preferably 0.01 to 0.5 parts by mass with respect to 100 parts by mass of the monomer mixture. When the content of the Lewis basic compound is within the above range, the monomer mixture reacts rapidly.

(Application)

The cured product according to the present embodiment may be a cured product of an (instant) adhesive. The use of the adhesive is as described above.

The cured product according to the present embodiment may be a coating material. The use of the coating material is as described above.

[Laminate According to First Embodiment]

The laminate according to the first embodiment of the present invention is a laminate containing the cured product according to the first embodiment and an adherend bonded to the cured product. The laminate according to the first embodiment of the present invention may be a laminate containing a cured product obtained by reacting the 1,1-dicyanoethylene-containing composition described above and an adherend bonded to the cured product. The laminated structure is not particularly limited, and the cured product may be formed on one adherend, or the cured product may be provided between two adherends. By having the cured product between two adherends, the adherends can be strongly bonded to each other.

The adherend used in the laminate of the present invention is not particularly limited, and examples thereof include a natural polymer, a synthetic polymer, a metal, ceramics, and a fabric.

Examples of the natural polymer include cellulose, natural rubber, starch, and modified resins thereof.

Examples of the synthetic polymer include polyolefin resins such as polyethylene, polypropylene, copolymers of ethylene and one or more $\alpha$-olefins having 3 to 20 carbon atoms (for example, propylene, 1-butene, 1-pentene, and 1-hexene), an ethylene-propylene-diene copolymer (EPDM), an ethylene-vinyl acetate copolymer, and an ethylene-acrylic acid copolymer, a polyurethane resin, a polyamide resin, a polyester resin, a polycarbonate resin, a vinyl chloride resin, an acrylonitrile-butadiene-styrene rubber, a natural rubber, a butadiene rubber, a styrene-butadiene rubber, and an acrylonitrile-butadiene rubber.

Examples of the metal include steel plates such as a stainless steel plate, a cold-rolled steel plate, and an alloyed galvanized steel plate, copper, aluminum, and magnesium alloys.

The thickness of the adherend constituting the laminate is not particularly limited. On the other hand, the thickness of the layer containing a cured product is preferably 0.01 to 2.0 mm, more preferably 0.015 to 1.5 mm, and still more preferably 0.02 to 1.2 mm, from the viewpoint of strongly bonding adherends to each other.

The method for producing a laminate is not particularly limited, and the laminate is preferably produced by the method for producing a laminate of the present invention including a bonding step of bonding a first adherend and a second adherend to each other via the cured product.

The method for bonding the first adherend and the second adherend to each other using the 1,1-dicyanoethylene-containing composition is not particularly limited; however, the first adherend and the second adherend can be bonded to each other, for example, by applying the 1,1-dicyanoethylene-containing composition to one adherend, overlaying the other adherend thereon, and curing the resultant.

The method for applying the 1,1-dicyanoethylene-containing composition to an adherend is not particularly limited, and examples thereof include a spin coating method, a spray coating method, a bar coating method, a knife coating method, a roll coating method, a roll knife coating method, a blade coating method, a die coating method, and a gravure coating method.

The amount of the 1,1-dicyanoethylene-containing composition applied to the adherend is not particularly limited, and is preferably 0.01 to 3.0 $\mu L/mm^2$, more preferably 0.05 to 2.5 $\mu L/mm^2$, and still more preferably 0.1 to 2.0 $\mu L/mm^2$. When the coating amount is equal to or more than the above lower limit value, it is possible to firmly bond adherends to each other. On the other hand, when the coating amount is equal to or less than the above upper limit value, it is possible to bond them with an appropriate amount.

[Second Embodiment]

[1, 1-Dicyanoethylene-Containing Composition According to Second Embodiment]

A 1,1-dicyanoethylene-containing composition according to a second embodiment of the present invention is a 1,1-dicyanoethylene-containing composition containing 1,1-dicyanoethylene, a phenol compound (A-2), and a specific compound (C). As will be described later in detail, the specific compound (C) is a compound for which a value of proton affinity (Epa) and a pKa value in the form of an aqueous solution are defined. When the 1,1-dicyanoethylene-containing

composition contains a specific phenol compound (A-2) and a specific compound (C), a 1,1-dicyanoethylene-containing composition having excellent storage stability can be provided. The phenol compound (A-2) may be one kind or a plurality of kinds. The specific compound (C) may be one kind or a plurality of kinds.

(1,1-Dicyanoethylene)

The 1, 1-dicyanoethylene contained in the 1,1-dicyanoethylene-containing composition according to the second embodiment is the same as the 1,1-dicyanoethylene contained in the 1,1-dicyanoethylene-containing composition according to the first embodiment.

(Phenol Compound (A-2))

The phenol compound (A-2) is represented by the following formula (II).

[Chem. 7]

OH, $R^{21}$, $R^{22}$, $R^{23}$, $R^{24}$, $R^{25}$

(II)

In the formula (II), $R^{21}$ and $R^{25}$ are a hydrogen atom or an o-substituent. The o-substituent refers to a substituent located at the o-position relative to a hydroxy group (the -OH group in the formula (II)) of the phenol compound. Here, in the second embodiment, unlike the first embodiment, a case where both $R^{21}$ and $R^{25}$ (that is, both of the two o-positions) are hydrogen atoms is allowed. This is because the hydroxy group of the phenol compound can be stabilized by containing the specific compound (C) in the 1,1-dicyanoethylene-containing composition according to the second embodiment. Then, it is considered that by stabilizing the hydroxy group of the phenol compound, the reaction with the coexisting 1,1-dicyanoethylene can be suppressed. The reaction between 1,1-dicyanoethylene and the phenol compound can be confirmed by thickening or solidification (precipitation) of the composition, as exemplified in the section of Examples.

In the formula (II), $R^{21}$ and $R^{25}$ are each independently a hydrogen atom or an o-substituent which is one selected from the group consisting of an alkyl group, a cycloalkyl group, an aryl group, an alkoxy group, a carboxy group, an ester group represented by -COOR$^{2a}$ (where $R^{2a}$ is an alkyl group having 1 to 25 carbon atoms), an acid anhydride group, an acyl group represented by -COR$^{2b}$ (where $R^{2b}$ is a hydrogen atom or an alkyl group having 1 to 25 carbon atoms), an acyloxy group represented by -OCOR$^{2c}$ (where $R^{2c}$ is an alkyl group having 1 to 25 carbon atoms), a hydroxy group, an amino group, a halogen atom, and a haloalkyl group. That is, in the formula (II), $R^{21}$ and $R^{25}$ are each independently a hydrogen atom, an alkyl group, a cycloalkyl group, an aryl group, an alkoxy group, a carboxy group, an ester group represented by -COOR$^{2a}$ (where $R^{2a}$ is an alkyl group having 1 to 25 carbon atoms), an acid anhydride group, an acyl group represented by -COR$^{2b}$ (where $R^{2b}$ is an alkyl group having 1 to 25 carbon atoms), an acyloxy group represented by -OCOR$^{2c}$ (where $R^{2c}$ is an alkyl group having 1 to 25 carbon atoms), a halogen atom, or a haloalkyl group.

In a preferable embodiment, both $R^{21}$ and $R^{25}$ are hydrogen atoms. Even in a case where both $R^{21}$ and $R^{25}$ in the phenol compound (A-2) are hydrogen atoms as described above, according to the second embodiment, it is possible to provide a 1,1-dicyanoethylene-containing composition having excellent storage stability.

The alkyl group that may be represented by $R^{21}$ and $R^{25}$ is preferably an alkyl group having 1 to 25 carbon atoms, and examples thereof include a methyl group, an ethyl group, a n-propyl group, an isopropyl group, a n-butyl group, an isobutyl group, a sec-butyl group, a tert-butyl group, a n-pentyl group, an isopentyl group, a neopentyl group, and a n-hexyl group.

The cycloalkyl group that may be represented by $R^{21}$ and $R^{25}$ is preferably a cycloalkyl group having 3 to 12 carbon atoms, and examples thereof include a cyclopropyl group, a cyclobutyl group, a cyclopentyl group, and a cyclohexyl group.

The aryl group that may be represented by $R^{21}$ and $R^{25}$ is preferably an aryl group having 6 to 25 carbon atoms, and examples thereof include a phenyl group, a tolyl group, a xylyl group, and a naphthyl group.

The alkoxy group that may be represented by $R^{21}$ and $R^{25}$ is preferably an alkoxy group having 1 to 25 carbon

atoms, and examples thereof include a methoxy group, an ethoxy group, a n-propoxy group, an isopropoxy group, a n-butoxy group, an isobutoxy group, a sec-butoxy group, and a tert-butoxy group.

In the ester group (-COOR$^{2a}$), the acyl group (-COR$^{2b}$), and the acyloxy group (-OCOR$^{2c}$) that may be represented by R$^{21}$ and R$^{25}$, examples of R$^{2a}$, R$^{2b}$ and R$^{2c}$ include a methyl group, an ethyl group, a n-propyl group, an isopropyl group, a n-butyl group, an isobutyl group, a sec-butyl group, a tert-butyl group, a n-pentyl group, an isopentyl group, a neopentyl group, a n-hexyl group, a heptyl group, an octyl group, a decyl group, a dodecyl group, and an octadecyl group.

Examples of the acid anhydride group that may be represented by R$^{21}$ and R$^{25}$ include acid anhydride groups derived from phthalic anhydride, maleic anhydride, trimellitic anhydride, pyromellitic anhydride, hexahydrophthalic anhydride, tetrahydrophthalic anhydride, methylnadic anhydride, nadic anhydride, glutaric anhydride, dimethylglutaric anhydride, diethylglutaric anhydride, succinic anhydride, methylhexahydrophthalic anhydride, and methyltetrahydrophthalic anhydride.

Examples of the halogen atom that may be represented by R$^{21}$ and R$^{25}$ include a fluorine atom, a chlorine atom, a bromine atom, and an iodine atom. The haloalkyl group that may be represented by R$^{21}$ and R$^{25}$ is preferably a haloalkyl group having 1 to 12 carbon atoms, and more preferably a haloalkyl group having 1 to 6 carbon atoms. Examples of the halogen atom constituting the haloalkyl group include a fluorine atom, a chlorine atom, a bromine atom, and an iodine atom.

Specific examples of the o-substituent that may be represented by R$^{21}$ and R$^{25}$ include a linear alkyl group having 1 to 25 carbon atoms, preferably 1 to 12 carbon atoms, and more preferably 1 to 6 carbon atoms, a branched alkyl group having 3 to 25 carbon atoms, preferably 3 to 12 carbon atoms, and more preferably 3 to 6 carbon atoms, a cycloalkyl group having 5 to 25 carbon atoms, preferably 5 to 12 carbon atoms, and more preferably 5 to 6 carbon atoms, an alkoxy group having 1 to 25 carbon atoms, preferably 1 to 12 carbon atoms, and more preferably 1 to 6 carbon atoms, and an aryl group having 6 to 25 carbon atoms, preferably 6 to 12 carbon atoms, and more preferably 6 carbon atoms. Among these, the o-substituent is preferably a linear alkyl group having 1 to 6 carbon atoms, a branched alkyl group having 3 to 6 carbon atoms, a cycloalkyl group having 5 to 12 carbon atoms, or an aryl group having 5 to 12 carbon atoms, more preferably a methyl group, an ethyl group, a n-propyl group, an isopropyl group, a n-butyl group, a sec-butyl group, a tert-butyl group, a n-pentyl group, a cyclohexyl group, or a phenyl group, still more preferably a tert-butyl group, a cyclohexyl group, or a phenyl group, and yet still more preferably a tert-butyl group.

In another preferable embodiment, one or both of R$^{21}$ and R$^{25}$ are each independently one selected from the group consisting of a branched alkyl group, a cycloalkyl group, and an aryl group. Preferably, one or both of R$^{21}$ and R$^{25}$ are each independently one selected from the group consisting of a branched alkyl group, a cycloalkyl group, and an aryl group. Examples of the branched alkyl group, the cycloalkyl group, and the aryl group include those described above.

In the formula (II), R$^{23}$ is a hydrogen atom or a p-substituent, and the p-substituent is the same as the o-substituent. When the p-substituent is the same as the o-substituent, it means that examples of the p-substituent are the same as those of the o-substituent. Therefore, the p-substituent and the o-substituent may be the same or different from each other. The p-substituent refers to a substituent located at the p-position relative to a hydroxy group (the -OH group in the formula (II)) of the phenol compound. That is, in the formula (II), R$^{23}$ is a hydrogen atom, an alkyl group, a cycloalkyl group, an aryl group, an alkoxy group, a carboxy group, an ester group represented by -COOR$^{2a}$ (where R$^{2a}$ is an alkyl group having 1 to 25 carbon atoms), an acid anhydride group, an acyl group represented by -COR$^{2b}$ (where R$^{2b}$ is an alkyl group having 1 to 25 carbon atoms), an acyloxy group represented by -OCOR$^{2c}$ (where R$^{2c}$ is an alkyl group having 1 to 25 carbon atoms), a halogen atom, or a haloalkyl group.

Specific examples of the p-substituent that may be represented by R$^{23}$ include a linear alkyl group having 1 to 25 carbon atoms, preferably 1 to 12 carbon atoms, and more preferably 1 to 6 carbon atoms, a branched alkyl group having 3 to 25 carbon atoms, preferably 3 to 12 carbon atoms, and more preferably 3 to 6 carbon atoms, a cycloalkyl group having 5 to 25 carbon atoms, preferably 5 to 12 carbon atoms, and more preferably 5 to 6 carbon atoms, an alkoxy group having 1 to 25 carbon atoms, preferably 1 to 12 carbon atoms, and more preferably 1 to 6 carbon atoms, a hydroxy group, and an aryl group having 6 to 25 carbon atoms, preferably 6 to 12 carbon atoms, and more preferably 6 carbon atoms. In one embodiment, R$^{23}$ is one p-substituent selected from a hydroxy group, a methyl group, and a methoxy group.

The p-substituent that may be represented by R$^{23}$ may be an aldehyde group. That is, R$^{26}$ in the acyl group (-COR$^{2b}$) may be a hydrogen atom. The p-substituent that may be represented by R$^{23}$ may be a combination of an amino group and -COR$^{2b}$. In this case, the p-substituent has an amide bond. The p-substituent that may be represented by R$^{23}$ may be a combination of -COR$^{2b}$, an amino group, and an alkyl group. In this case, the p-substituent has an amide bond.

In the formula (II), R$^{22}$ and R$^{24}$ are each independently a hydrogen atom or a m-substituent, and the m-substituent is the same as the p-substituent. When the m-substituent is the same as the p-substituent, it means that examples of the m-substituent are the same as those of the p-substituent. Therefore, the m-substituent and the p-substituent may be the same or different from each other. The m-substituent refers to a substituent located at the m-position relative to a hydroxy group (the -OH group in the formula (II)) of the phenol compound. That is, in the formula (II), R$^{22}$ and R$^{24}$ are each independently a hydrogen atom, an alkyl group, a cycloalkyl group, an aryl group, an alkoxy group, a

carboxy group, an ester group represented by -COOR$^{2a}$ (where R$^{2a}$ is an alkyl group having 1 to 25 carbon atoms), an acid anhydride group, an acyl group represented by -COR$^{2b}$ (where R$^{2b}$ is an alkyl group having 1 to 25 carbon atoms), an acyloxy group represented by -OCOR$^{2c}$ (where R$^{2c}$ is an alkyl group having 1 to 25 carbon atoms), a halogen atom, or a haloalkyl group. R$^{22}$ and R$^{24}$ are each independently preferably a hydrogen atom, or one selected from the group consisting of a linear alkyl group having 1 to 25 carbon atoms, preferably 1 to 12 carbon atoms, and more preferably 1 to 6 carbon atoms, and a branched alkyl group having 3 to 25 carbon atoms, preferably 3 to 12 carbon atoms, and more preferably 3 to 6 carbon atoms, and more preferably a hydrogen atom.

Specific examples of the phenol compound (A-2) include hydroquinone, p-methylphenol, p-methoxyphenol, tert-butylhydroquinone, 2,6-di-tert-butyl-4-methylphenol, 2,6-di-tert-butylphenol, pentaerythritol tetrakis[3-(3,5-di-tert-butyl-4-hydroxyphenyl)propionate], octadecyl 3-(3,5-di-tert-butyl-4-hydroxyphenyl)propionate, 2,4-di-tert-butylphenol, 2,2'-methylenebis(4-methyl-6-tert-butylphenol), 2,2'-methylenebis-(4-ethyl-6-tert-butylphenol) (YOSHINOX 425), 4,4'-butylidenebis-(6-tert-butyl-3-methylphenol) (YOSHINOX BB), 2,2'-methylenebis[6-tert-butyl-4-methylphenol] (SUMILIZER MDP-S), 1,1,3-tris(2-methyl-4-hydroxy-5-tert-butylphenyl)butane (ADK STAB AO-30), 4,4'-butylidenebis(6-tert-butyl-m-cresol) (ADK STAB AO-40), octadecyl 3-(3,5-di-tert-butyl-4-hydroxyphenyl)propionate (ADK STAB AO-50), 2,2'-dimethyl-2,2'-(2,4,8,10-tetraoxaspiro[5.5]undecane-3,9-diyl)dipropane-1,1'-diyl=bis[3-(3-tert-butyl-4-hydroxy-5-methylphenyl)propanoate] (ADK STAB AO-80), 1,3,5-trimethyl-2,4,6-tris(3,5-di-tert-butyl-4-hydroxybenzyl)benzene (ADK STAB AO-330), 4,4'-thiobis[3-methyl-6-(tert-butyl)phenol] (SUMILIZER WX-R), 2,6-bis[(2-hydroxy-5-methylphenyl)methyl]-4-methylphenol, 2,4,6-tris(3', 5'-di-tert-butyl-4'-hydroxybenzyl)mesitylene, 1'-hydroxy[2,2'-ethylidenebis [4,6-bis(1,1-dimethylpropyl)benzene]]-1-yl acrylate (SUMILIZER GS), 2-tert-butyl-4-methyl-6-(2-hydroxy-3-tert-butyl-5-methylbenzyl)phenyl acrylate (SUMILIZER GM), and 2-tert-butyl-6-methyl-4-{3-[(2,4,8,10-tetra-tert-butyldibenzo [d,f][1,3,2]dioxaphosphepin-6-yl)oxy] propyl}phenol (SUMILIZER GP). In a preferable embodiment, the phenol compound (A-2) is at least one selected from 2,6-di-tert-butylphenol, 2,6-di-tert-butyl-4-methylphenol, pentaerythritol tetrakis [3-(3,5-di-tert-butyl-4-hydroxyphenyl)propionate], octadecyl 3-(3,5-di-tert-butyl-4-hydroxyphenyl)propionate, and 2,4-di-tert-butylphenol. In a more preferable embodiment, the phenol compound (A-2) is at least one selected from 2,6-di-tert-butylphenol, 2,6-di-tert-butyl-4-methylphenol, pentaerythritol tetrakis[3-(3,5-di-tert-butyl-4-hydroxyphenyl)propionate], and octadecyl 3-(3,5-di-tert-butyl-4-hydroxyphenyl)propionate.

From the viewpoint of suppressing coloration of the 1,1-dicyanoethylene-containing composition, the phenol compound (A-2) preferably has, as a p-substituent, a substituent other than a hydrogen atom, and preferably has a substituent other than a hydrogen atom and a halogen atom.

(Specific Compound (C))

The 1,1-dicyanoethylene-containing composition according to the second embodiment contains the specific compound (C) as described above. The specific compound (C) is a compound having a proton affinity (Epa) of 780 kJ/mol or less and a pKa of 5 or less in the form of an aqueous solution.

(Proton Affinity (Epa) and pKa in the Form of Aqueous Solution)

As a value of the proton affinity (Epa), a literature value can be used. Examples of the literature include NPL 2 (Edward P. H., Dharon G. L., Journal of Physical and Chemical Reference Data 1998, 27, 413-656). Epa of the specific compound (C) is preferably 775 kJ/mol or less, more preferably 770 kJ/mol or less, and still more preferably 765 kJ/mol or less. The lower limit of Epa is preferably higher than Epa (691 kJ/mol) of water, and may be, for example, more than 691 kJ/mol or 695 kJ/mol or more.

As a value of pKa in the form of an aqueous solution, it is possible to refer to NPL 3 (pKa table by D. H. Ripin and D. A. Evans; available from https://depts.washington.edu/eooptic/linkfiles/evans_pKa_table.pdf). The pKa of the specific compound (C) in the form of an aqueous solution is preferably 4 or less, more preferably 3 or less, and still more preferably 2 or less. The lower limit of the pKa is not particularly limited, and may be, for example, -5 or more or -4 or more.

Examples of the compound having an Epa of 780 kJ/mol or less and a pKa of 5 or less in the form of an aqueous solution include methanesulfonic acid (Epa: 761.3 kJ/mol, pKa: -2.6), sulfuric acid (Epa: 699. 4 kJ/mol, pKa: -3.0), sulfur dioxide (Epa: 672.3 kJ/mol, pKa as sulfurous acid: 1.9), and nitric acid (Epa: 751.4 kJ/mol, pKa: -1.3). In one embodiment, methanesulfonic acid is used as the specific compound (C). In another embodiment, at least one selected from the group consisting of methanesulfonic acid, sulfuric acid, and sulfur dioxide is used as the specific compound (C).

(Component (B): Polymerizable Monomer)

The 1,1-dicyanoethylene-containing composition according to the second embodiment may further contain a polymerizable monomer (B). The polymerizable monomer (B) excludes 1,1-dicyanoethylene. The polymerizable monomer (B) is preferably reactive with 1,1-dicyanoethylene. The polymerizable monomer (B) may be one kind or a plurality of

kinds. The polymerizable monomer (B) may be a radically polymerizable monomer or an anionically polymerizable monomer. Examples of the polymerizable monomer (B) and a method for producing the polymerizable monomer (B) are as described in the first embodiment.

(Other Component)

The 1,1-dicyanoethylene-containing composition according to the second embodiment may contain one or more other components.

Examples of such other components include acid anhydrides, Lewis acidic compounds, thickeners, dehydrating agents, radical polymerization inhibitors (excluding the above-described phenolic compounds), plasticizers, pigments, organic solvents, rubbers, organic fillers, and inorganic fillers. The other components can be used in such an amount that the expected effect of the present invention is not impaired. The acid anhydride may also function as a dehydrating agent. The acid anhydride may also function as a dehydrating agent. The rubber may also function as an organic filler. The inorganic filler may also function as a thickener.

(Content of Each Component)

The total amount of the monomers (1,1-dicyanoethylene and the polymerizable monomer (B) used as required) is preferably 70% by mass or more, more preferably 80% by mass or more, and still more preferably 90% by mass or more, when the entire 1,1-dicyanoethylene-containing composition according to the second embodiment is taken as 100% by mass, from the viewpoint of achieving the expected physical properties. When the polymerizable monomer (B) is used, the mass ratio of 1,1-dicyanoethylene to the polymerizable monomer (B) is not particularly limited, and may be 99:1 to 1:99, 80:20 to 20:80, or 70:30 to 30:70.

The phenol compound (A-2) is used in an amount of preferably 10 parts by mass or less, more preferably 5.0 parts by mass or less, still more preferably 2.0 parts by mass or less, and yet still more preferably 1.5 parts by mass or less, when the total amount of the monomers (1,1-dicyanoethylene and the polymerizable monomer (B) used as required) is taken as 100 parts by mass. From the viewpoint of enhancing the storage stability of the 1,1-dicyanoethylene-containing composition, the phenol compound (A-2) is used in an amount of preferably 0.0005 parts by mass or more, more preferably 0.001 parts by mass or more, and still more preferably 0.005 parts by mass or more when the total amount of the monomers is taken as 100 parts by mass.

The content of the phenol compound (A-2) is preferably 0.00003% by mass or more, more preferably 0.00006% by mass or more, and still more preferably 0.00009% by mass or more, and preferably 10% by mass or less, more preferably 5.0% by mass or less, still more preferably 2.0% by mass or less, and yet still more preferably 1.5% by mass or less, when the entire 1,1-dicyanoethylene-containing composition is taken as 100% by mass, from the viewpoint of enhancing the storage stability of the 1,1-dicyanoethylene-containing composition.

The specific compound (C) is used in an amount of preferably 10 parts by mass or less, more preferably 5.0 parts by mass or less, still more preferably 2.0 parts by mass or less, and yet still more preferably 1.0 parts by mass or less, when the total amount of the monomers (1,1-dicyanoethylene and the polymerizable monomer (B) used as required) is taken as 100 parts by mass. From the viewpoint of enhancing the storage stability of the 1,1-dicyanoethylene-containing composition, the amount of the specific compound (C) is used in an amount of preferably 0.0005 parts by mass or more, more preferably 0.001 parts by mass or more, and still more preferably 0.005 parts by mass or more when the total amount of the monomers is taken as 100 parts by mass.

The content of the specific compound (C) is preferably 0.0005% by mass or more, more preferably 0.0009% by mass or more, and still more preferably 0.004% by mass or more, and preferably 10% by mass or less, more preferably 5.0% by mass or less, still more preferably 2.0% by mass or less, and yet still more preferably 1.0% by mass or less, when the entire 1,1-dicyanoethylene-containing composition is taken as 100% by mass, from the viewpoint of enhancing the storage stability of the 1,1-dicyanoethylene-containing composition.

The mass ratio of the specific compound (C) to the phenol compound (A-2) is not limited as long as the expected effect of the present invention is exhibited, and may be, for example, in the range of 1:10000 to 10000:1, preferably in the range of 1:1000 to 1000:1, more preferably in the range of 1:500 to 500:1, and still more preferably in the range of 1:250 to 250:1. In a preferable embodiment, the content (parts by mass) of the specific compound (C) is equal to or less than the content (parts by mass) of the phenol compound (A-2).

(Application)

The 1,1-dicyanoethylene-containing composition according to the present embodiment can be used as an (instant) adhesive. The application of such an adhesive is as described in the first embodiment.

An instant adhesive containing ethyl 2-cyanoacrylate as a main raw material is widely known, but ethyl 2-

cyanoacrylate has poor moisture resistance, and the instant adhesive is inferior in moisture resistance. On the other hand, 1,1-dicyanoethylene has excellent moisture resistance, and an adhesive using the 1,1-dicyanoethylene-containing composition according to the present embodiment has excellent moisture resistance.

The 1,1-dicyanoethylene-containing composition according to the present embodiment can also be used as a coating material. The application of such a coating material is as described in the first embodiment.

[Cured Product According to Second Embodiment]

The cured product according to the second embodiment of the present invention is a cured product obtained by reacting 1,1-dicyanoethylene with a Lewis basic compound in the presence of a phenol compound (A-2) represented by the following formula (II) and a compound (C) having a proton affinity (Epa) of 780 kJ/mol or less and a pKa of 5 or less in the form of an aqueous solution:

[Chem. 8]

(II)

in which $R^{21}$ and $R^{25}$ are each independently a hydrogen atom or an o-substituent which is one selected from the group consisting of an alkyl group, a cycloalkyl group, an aryl group, an alkoxy group, a carboxy group, an ester group represented by $-COOR^{2a}$ (where $R^{2a}$ is an alkyl group having 1 to 25 carbon atoms), an acid anhydride group, an acyl group represented by $-COR^{2b}$ (where $R^{2b}$ is a hydrogen atom or an alkyl group having 1 to 25 carbon atoms), an acyloxy group represented by $-OCOR^{2c}$ (where $R^{2c}$ is an alkyl group having 1 to 25 carbon atoms), a hydroxy group, an amino group, a halogen atom, and a haloalkyl group; that is, in the formula (II), $R^{21}$ and $R^{25}$ are each independently a hydrogen atom, an alkyl group, a cycloalkyl group, an aryl group, an alkoxy group, a carboxy group, an ester group represented by $-COOR^{2a}$ (where $R^{2a}$ is an alkyl group having 1 to 25 carbon atoms), an acid anhydride group, an acyl group represented by $-COR^{2b}$ (where $R^{2b}$ is an alkyl group having 1 to 25 carbon atoms), an acyloxy group represented by $-OCOR^{2c}$ (where $R^{2c}$ is an alkyl group having 1 to 25 carbon atoms), a halogen atom, or a haloalkyl group.

$R^{23}$ is each independently a hydrogen atom or a p-substituent, and the p-substituent is the same as the o-substituent; and that is, $R^{23}$ is a hydrogen atom, an alkyl group, a cycloalkyl group, an aryl group, an alkoxy group, a carboxy group, an ester group represented by $-COOR^{2a}$ (where $R^{2a}$ is an alkyl group having 1 to 25 carbon atoms), an acid anhydride group, an acyl group represented by $-COR^{2b}$ (where $R^{2b}$ is an alkyl group having 1 to 25 carbon atoms), an acyloxy group represented by $-OCOR^{2c}$ (where $R^{2c}$ is an alkyl group having 1 to 25 carbon atoms), a halogen atom, or a haloalkyl group.

$R^{22}$ and $R^{24}$ are each independently a hydrogen atom or a m-substituent, and the m-substituent is the same as the p-substituent. That is, $R^{22}$ and $R^{24}$ are each independently a hydrogen atom, an alkyl group, a cycloalkyl group, an aryl group, an alkoxy group, a carboxy group, an ester group represented by $-COOR^{2a}$ (where $R^{2a}$ is an alkyl group having 1 to 25 carbon atoms), an acid anhydride group, an acyl group represented by $-COR^{2b}$ (where $R^{2b}$ is an alkyl group having 1 to 25 carbon atoms), an acyloxy group represented by $-OCOR^{2c}$ (where $R^{2c}$ is an alkyl group having 1 to 25 carbon atoms), a halogen atom, or a haloalkyl group.

The cured product according to the second embodiment of the present invention may be a cured product obtained by reacting the 1,1-dicyanoethylene-containing composition described above.

(Monomer Mixture)

1,1-dicyanoethylene may or may not constitute a monomer mixture together with the polymerizable monomer (B). In the case of a monomer mixture, the 1,1-dicyanoethylene, the phenol compound (A-2) represented by the formula (II), and the specific compound (C) are respectively the same as the 1,1-dicyanoethylene, the phenol compound (A-2)

represented by the formula (II), and the specific compound (C) contained in the 1,1-dicyanoethylene-containing composition according to the second embodiment. The polymerizable monomer (B) that can be contained in the monomer mixture is the same as the polymerizable monomer (B) that can be contained in the 1,1-dicyanoethylene-containing composition according to the second embodiment. The monomer mixture may contain one or more other components. Such other components are the same as the other components that can be contained in the 1,1-dicyanoethylene-containing composition in the second embodiment. In one embodiment, the cured product is a cured product obtained by reacting 1,1-dicyanoethylene, a polymerizable monomer (B), and a Lewis basic compound in the presence of a phenol compound (A-2) represented by the formula (II).

(Lewis Basic Compound)

The Lewis basic compound usually functions as a polymerization catalyst for 1,1-dicyanoethylene or the monomer mixture. Examples of the Lewis basic compound include water, alcohols, and alkylamines. Examples of alcohols and alkylamines are the same as those described in the first embodiment. Among these, in one embodiment, water is used as the Lewis basic compound.

(Other Additives)

The cured product according to the present embodiment may contain, as other additives, at least one selected from the group consisting of thickeners (e.g., organic thickeners, inorganic thickeners such as silica), dehydrating agents (e.g., carboxylic acid anhydrides such as acetic anhydride, cyclic sulfonic acid esters such as propane sultone, and phosphoric acid anhydrides such as diphosphorus pentoxide), radical polymerization inhibitors (e.g., stable radical compounds, and metal salts), plasticizers (e.g., ester compounds such as phthalic acid esters and adipic acid esters), rubbers (e.g., natural rubbers, styrene-butadiene rubbers, hydrogenated styrene-butadiene rubbers, acrylic rubbers, nitrile rubbers, and hydrogenated nitrile rubbers), pigments, and fillers (e.g., inorganic fillers and organic fillers) in an amount that does not excessively impair the expected effect of the present invention. Specific examples of the organic thickener include polymer compounds such as an ethylene-vinyl acetate copolymer, a methyl (meth)acrylate resin, a polystyrene resin, a (modified) cellulose resin, and an acrylonitrile resin. Specific examples of the stable radical compound include TEMPO (2,2,6,6-tetramethylpiperidine 1-oxyl). The inorganic thickener may also function as an inorganic filler. The above-mentioned phenolic compound is excluded from the radical polymerization inhibitor. The rubber may also function as an organic filler. Further, a hardening accelerator such as a polyethylene glycol derivative, crown ether, and calixarene can be added for the purpose of improving the bonding speed. Furthermore, a filler, an elastomer, a thixotropy imparting agent, an adhesion imparting agent, a crosslinking agent, a fragrance, and the like may be added according to the purpose.

(Production Method of Cured Product)

The production method of the cured product is not particularly limited. For example, a cured product can be obtained by mixing the monomer mixture with the Lewis basic compound at room temperature (23°C).

The amount of the Lewis basic compound is not particularly limited, and is preferably 0.001 to 1.0 parts by mass, and more preferably 0.01 to 0.5 parts by mass with respect to 100 parts by mass of the monomer mixture. When the content of the Lewis basic compound is within the above range, the monomer mixture reacts rapidly.

(Application)

The cured product according to the present embodiment may be a cured product of an (instant) adhesive. The use of the adhesive is as described above.

The cured product according to the present embodiment may be a coating material. The use of the coating material is as described above.

[Laminate According to Second Embodiment]

The laminate according to the second embodiment of the present invention is a laminate containing the cured product according to the second embodiment and an adherend bonded to the cured product. The laminate according to the second embodiment of the present invention may be a laminate containing a cured product obtained by reacting the 1,1-dicyanoethylene-containing composition described above and an adherend bonded to the cured product. The laminated structure, the adherend, and the method for producing the laminate are the same as described in the first embodiment.

Examples

Hereinafter, the present invention will be described in detail by Examples, but the present invention is not limited to these Examples.

[First Examples]

[Component]

The components used in Examples and Comparative Examples were as follows.

<1,1-Dicyanoethylene>

1,1-Dicyanoethylene: 1,1,3,3-Tetracyanopropane was synthesized in a yield of 73% from malononitrile by the production method described in J. Am. Chem. Soc., 1989, 111, 9078-9081. The obtained crystalline 1,1,3,3-tetracyanopropane and diphosphorus pentoxide were mixed and thermally decomposed at 180°C to obtain a crude product of 1,1-dicyanoethylene (yield 60%).
The crude product was purified by vacuum distillation (480 Pa) to obtain 1,1-dicyanoethylene with a purity of 99%.

<Component (B): Polymerizable Monomer>

(B1): Ethyl 2-cyanoacrylate (manufactured by Sigma-Aldrich Co., LLC)

<Component (A-1): o-Substituted Phenol Compound>

(A11): 2,6-Di-tert-butyl-4-methylphenol (manufactured by FUJIFILM Wako Pure Chemical Corporation) (Taft value of o-position tert-butyl group: -1.54)
(A12): 2,6-Di-tert-butylphenol (manufactured by Tokyo Chemical Industry Co., Ltd.) (Taft value of tert-butyl group: -1.54)
(A13): "Irganox (registered trademark) 1010" manufactured by BASF SE (pentaerythritol tetrakis[3-(3,5-di-tert-butyl-4-hydroxyphenyl)propionate]) (Taft value of tert-butyl group: -1.54)
(A14): "Irganox (registered trademark) 1076" manufactured by FUJIFILM Wako Pure Chemical Corporation (octadecyl 3-(3,5-di-tert-butyl-4-hydroxyphenyl)propionate) (Taft value of tert-butyl group: -1.54)
(A15): 2,4-Di-tert-butylphenol (manufactured by Tokyo Chemical Industry Co., Ltd.) (Taft value of tert-butyl group: -1.54, Taft value of hydrogen atom: 1.24)

As the Taft value mentioned above, the Taft value based on the literature value for the substituent at the o-position relative to the hydroxy group of phenol was adopted (Literature: NPL 1).

<Comparative Component (A-1)>

(A'11): N-Nitroso-N-phenylhydroxylamine aluminum ("Q-1301" manufactured by FUJIFILM Wako Pure Chemical Corporation)
(A'12): p-Benzoquinone (manufactured by NACALAI TESQUE, INC.)
(A'13): Hydroquinone (manufactured by FUJIFILM Wako Pure Chemical Corporation)
(A'14): p-Methylphenol (manufactured by Tokyo Chemical Industry Co., Ltd.)
(A'15): p-Methoxyphenol (manufactured by Tokyo Chemical Industry Co., Ltd.)

<Other Component>

Methanesulfonic acid: manufactured by Tokyo Chemical Industry Co., Ltd.

[Evaluation Methods]

The liquid 1,1-dicyanoethylene-containing compositions obtained in Examples and Comparative Examples were evaluated as samples in the following manner.

<Storage Stability>

(Storage Test)

A sample of 1 mL was placed in a container made of HDPE of 10 mL and tightly sealed, the container was marked at a position corresponding to the sample liquid surface on the side surface of the container, and five of such containers were prepared. The state of the sample in each container immediately before the start of the storage test was visually observed. Thereafter, the five containers were allowed to stand in an environment with a humidity of 50% at 25°C for 24 hours, at 25°C for 48 hours, at 50°C for 1 hour, at 80°C for 1 hour, and at 80°C for 3 hours, respectively. In this way, the storage test was performed.

(Observation)

Thereafter, each container was inverted, and the state of the sample in the container was visually observed 5 seconds after the completion of the inversion.

The observation results were classified into the following three grades.

"Liquid": 90% by volume or more of the sample in the container exceeded the mark in a side view.
"Thickening": 10% by volume or more of the sample in the container exceeded the mark in a side view, but 90% by volume or more did not exceed the mark in a side view. This meant that the fluidity of the sample decreased as compared with that immediately before the start of the storage test (0 minutes).
"Solidification": Less than 10% by volume of the sample in the container exceeded the mark in a side view.

(Evaluation)

Subsequently, based on the observation results of the samples in the five containers in five grades after the storage test, evaluation was performed according to the following criteria.

"A": The total number of "thickening" and "solidification" instances was 0.
"B": The total number of "thickening" and "solidification" instances was 1.
"C": The total number of "thickening" and "solidification" instances was either 2 or 3. Among these, "C1" had 1 "solidification" instance, "C2" had 2 "solidification" instances, and "C3" had 3 "solidification" instances.
"D": The total number of "thickening" and "solidification" instances was 4.
"E": The total number of "thickening" and "solidification" instances was 5.

<Colorability>

(Observation)

During the <Storage Test>, the color of the sample inside the container was visually observed at the time of visual inspection. The observed sample color was classified into three levels: "colorless", "light yellow", and "yellow". Note that "yellow" indicates a darker color than "light yellow".

(Evaluation)

Subsequently, based on the six observation results (including observation result at 0 minutes) of the samples in the five containers after the storage test, evaluation was performed according to the following criteria.

"A": The total number of "light yellow" and "yellow" was 0.
"B": The number of "light yellow" was 1 to 3, and the number of "yellow" was 0.
"C": The number of "light yellow" was 4 or 5, and the number of "yellow" was 0.
"D": The number of "yellow" was 1 to 4.
"E": The number of "Yellow" was 5 or more.

<Example 1-1>

Under nitrogen atmosphere, a 1,1-dicyanoethylene-containing composition 1-1 was prepared by mixing 100 parts by mass of 1,1-dicyanoethylene with 0.0001 parts by mass of 2,6-di-tert-butyl-4-methylphenol (component (A11)).
Using the prepared 1,1-dicyanoethylene-containing composition 1-1, the above-described evaluations of storage stability and colorability were carried out. The results are shown in Table 1.

<Examples 1-2 to 1-22 and Comparative Examples 1-1 to 1-12>

1,1-Dicyanoethylene-containing compositions 1-2 to 1-22 and 1'-1 to 1'-12 were prepared according to the formulations shown in Table 1, Table 2, and Table 3. Multiple components were mixed simultaneously.
Using the prepared 1,1-dicyanoethylene-containing compositions, evaluations of storage stability and colorability were carried out in the same manner as in Example 1-1. The results are shown in Tables 1 to 3.

[Table 1]

Table 1

| | | | Example 1-1 | Example 1-2 | Example 1-3 | Example 1-4 | Example 1-5 |
|---|---|---|---|---|---|---|---|
| 1,1-Dicyanoethylene-containing composition | | | 1-1 | 1-2 | 1-3 | 1-4 | 1-5 |
| Composition | 1,1-Dicyanoethylene [parts by mass] | 1,1-Dicyanoethylene | 100 | 100 | 100 | 100 | 100 |
| | Component (B) [parts by mass] | (B1) | 0 | 0 | 0 | 0 | 0 |
| | | (B2) | 0 | 0 | 0 | 0 | 0 |
| | | (B3) | 0 | 0 | 0 | 0 | 0 |
| | | (B4) | 0 | 0 | 0 | 0 | 0 |
| | Component (A-1) [parts by mass] | (A11) | 0.0001 | 0.001 | 0.1 | 1 | 1 |
| | | (A12) | 0 | 0 | 0 | 0 | 0 |
| | | (A13) | 0 | 0 | 0 | 0 | 0 |
| | | (A14) | 0 | 0 | 0 | 0 | 0 |
| | | (A15) | 0 | 0 | 0 | 0 | 0 |
| | Comparative component (A-1) [parts by mass] | (A'11) | 0 | 0 | 0 | 0 | 0 |
| | | (A'12) | 0 | 0 | 0 | 0 | 0 |
| | | (A'13) | 0 | 0 | 0 | 0 | 0 |
| | | (A'14) | 0 | 0 | 0 | 0 | 0 |
| | | (A'15) | 0 | 0 | 0 | 0 | 0 |
| | Other component [parts by mass] | Methanesulfonic acid | 0 | 0 | 0 | 0 | 0.01 |
| Evaluation | Storage stability | 25°C, 0 minutes | Liquid | Liquid | Liquid | Liquid | Liquid |
| | | 25°C, 24 hours | Liquid | Liquid | Liquid | Liquid | Liquid |
| | | 25°C, 48 hours | Liquid | Liquid | Liquid | Liquid | Liquid |
| | | 50°C, 1 hour | Liquid | Liquid | Liquid | Liquid | Liquid |
| | | 80°C, 1 hour | Liquid | Liquid | Liquid | Liquid | Liquid |
| | | 80°C, 3 hours | Solidification | Solidification | Solidification | Solidification | Liquid |
| | | Evaluation | B | B | B | B | A |
| | Colorability | 25°C, 0 minutes | Colorless | Colorless | Colorless | Colorless | Colorless |
| | | 25°C, 24 hours | Colorless | Colorless | Colorless | Colorless | Colorless |
| | | 25°C, 48 hours | Colorless | Colorless | Colorless | Colorless | Colorless |
| | | 50°C, 1 hour | Colorless | Colorless | Colorless | Colorless | Colorless |
| | | 80°C, 1 hour | Colorless | Colorless | Colorless | Light yellow | Colorless |
| | | 80°C, 3 hours | Light yellow | Light yellow | Light yellow | Light yellow | Colorless |
| | | Evaluation | B | B | B | B | A |

Table 1 (continued)

| | | | Example 1-6 | Example 1-7 | Example 1-8 | Example 1-9 | Example 1-10 |
|---|---|---|---|---|---|---|---|
| 1,1-Dicyanoethylene-containing composition | | | 1-6 | 1-7 | 1-8 | 1-9 | 1-10 |
| Composition | 1,1-Dicyanoethylene [parts by mass] | 1,1-Dicyanoethylene | 100 | 100 | 100 | 90 | 50 |
| | Component (B) [parts by mass] | (B1) | 0 | 0 | 0 | 10 | 50 |
| | | (B2) | 0 | 0 | 0 | 0 | 0 |
| | | (B3) | 0 | 0 | 0 | 0 | 0 |
| | | (B4) | 0 | 0 | 0 | 0 | 0 |
| | Component (A-1) [parts by mass] | (A11) | 1 | 0.1 | 0.01 | 0.0001 | 0.0001 |
| | | (A12) | 0 | 0 | 0 | 0 | 0 |
| | | (A13) | 0 | 0 | 0 | 0 | 0 |
| | | (A14) | 0 | 0 | 0 | 0 | 0 |
| | | (A15) | 0 | 0 | 0 | 0 | 0 |
| | Comparative component (A-1) [parts by mass] | (A'11) | 0 | 0 | 0 | 0 | 0 |
| | | (A'12) | 0 | 0 | 0 | 0 | 0 |
| | | (A'13) | 0 | 0 | 0 | 0 | 0 |
| | | (A'14) | 0 | 0 | 0 | 0 | 0 |
| | | (A'15) | 0 | 0 | 0 | 0 | 0 |
| | Other component [parts by mass] | Methanesulfonic acid | 0.1 | 0.01 | 0.01 | 0.01 | 0.01 |
| Evaluation | Storage stability | 25°C, 0 minutes | Liquid | Liquid | Liquid | Liquid | Liquid |
| | | 25°C, 24 hours | Liquid | Liquid | Liquid | Liquid | Liquid |
| | | 25°C, 48 hours | Liquid | Liquid | Liquid | Liquid | Liquid |
| | | 50°C, 1 hour | Liquid | Liquid | Liquid | Liquid | Liquid |
| | | 80°C, 1 hour | Liquid | Liquid | Liquid | Liquid | Liquid |
| | | 80°C, 3 hours | Liquid | Liquid | Liquid | Liquid | Liquid |
| | | Evaluation | A | A | A | A | A |
| | Colorability | 25°C, 0 minutes | Colorless | Colorless | Colorless | Colorless | Colorless |
| | | 25°C, 24 hours | Colorless | Colorless | Colorless | Colorless | Colorless |
| | | 25°C, 48 hours | Colorless | Colorless | Colorless | Colorless | Colorless |
| | | 50°C, 1 hour | Colorless | Colorless | Colorless | Colorless | Colorless |
| | | 80°C, 1 hour | Colorless | Colorless | Colorless | Colorless | Colorless |
| | | 80°C, 3 hours | Colorless | Colorless | Colorless | Colorless | Colorless |
| | | Evaluation | A | A | A | A | A |

Table 1 (continued)

| | | | Example 1-11 | Example 1-12 | Example 1-13 | Example 1-14 |
|---|---|---|---|---|---|---|
| 1,1-Dicyanoethylene-containing composition | | | 1-11 | 1-12 | 1-13 | 1-14 |
| Composition | 1,1-Dicyanoethylene [parts by mass] | 1,1-Dicyanoethylene | 30 | 50 | 50 | 50 |
| | Component (B) [parts by mass] | (B1) | 70 | 0 | 0 | 0 |
| | | (B2) | 0 | 50 | 0 | 0 |
| | | (B3) | 0 | 0 | 50 | 0 |
| | | (B4) | 0 | 0 | 0 | 50 |
| | Component (A-1) [parts by mass] | (A11) | 0.0001 | 0.0001 | 0.0001 | 0.0001 |
| | | (A12) | 0 | 0 | 0 | 0 |
| | | (A13) | 0 | 0 | 0 | 0 |
| | | (A14) | 0 | 0 | 0 | 0 |
| | | (A15) | 0 | 0 | 0 | 0 |
| | Comparative component (A-1) [parts by mass] | (A'11) | 0 | 0 | 0 | 0 |
| | | (A'12) | 0 | 0 | 0 | 0 |
| | | (A'13) | 0 | 0 | 0 | 0 |
| | | (A'14) | 0 | 0 | 0 | 0 |
| | | (A'15) | 0 | 0 | 0 | 0 |
| | Other component [parts by mass] | Methanesulfonic acid | 0.01 | 0.01 | 0.01 | 0.01 |
| Evaluation | Storage stability | 25°C, 0 minutes | Liquid | Liquid | Liquid | Liquid |
| | | 25°C, 24 hours | Liquid | Liquid | Liquid | Liquid |
| | | 25°C, 48 hours | Liquid | Liquid | Liquid | Liquid |
| | | 50°C, 1 hour | Liquid | Liquid | Liquid | Liquid |
| | | 80°C, 1 hour | Liquid | Liquid | Liquid | Liquid |
| | | 80°C, 3 hours | Liquid | Liquid | Liquid | Liquid |
| | | Evaluation | A | A | A | A |
| | Colorability | 25°C, 0 minutes | Colorless | Colorless | Colorless | Colorless |
| | | 25°C, 24 hours | Colorless | Colorless | Colorless | Colorless |
| | | 25°C, 48 hours | Colorless | Colorless | Colorless | Colorless |
| | | 50°C, 1 hour | Colorless | Colorless | Colorless | Colorless |
| | | 80°C, 1 hour | Colorless | Colorless | Colorless | Colorless |
| | | 80°C, 3 hours | Colorless | Colorless | Colorless | Colorless |
| | | Evaluation | A | A | A | A |

[Table 2]

Table 2

| | | | Example 1-15 | Example 1-16 | Example 1-17 | Example 1-18 |
|---|---|---|---|---|---|---|
| 1,1-Dicyanoethylene-containing composition | | | 1-15 | 1-16 | 1-17 | 1-18 |
| Composition | 1,1-Dicyanoethylene [parts by mass] | 1,1-Dicyanoethylene | 100 | 100 | 100 | 100 |
| | Component (B) [parts by mass] | (B1) | 0 | 0 | 0 | 0 |
| | Component (A-1) [parts by mass] | (A11) | 0 | 0 | 0 | 0 |
| | | (A12) | 0.1 | 0.1 | 0 | 0 |
| | | (A13) | 0 | 0 | 0.1 | 0.1 |
| | | (A14) | 0 | 0 | 0 | 0 |
| | | (A15) | 0 | 0 | 0 | 0 |
| | Comparative component (A-1) [parts by mass] | (A'11) | 0 | 0 | 0 | 0 |
| | | (A'12) | 0 | 0 | 0 | 0 |
| | | (A'13) | 0 | 0 | 0 | 0 |
| | | (A'14) | 0 | 0 | 0 | 0 |
| | | (A'15) | 0 | 0 | 0 | 0 |
| | Other component [parts by mass] | Methanesulfonic acid | 0 | 0.01 | 0 | 0.01 |
| Evaluation | Storage stability | 25°C, 0 minutes | Liquid | Liquid | Liquid | Liquid |
| | | 25°C, 24 hours | Liquid | Liquid | Liquid | Liquid |
| | | 25°C, 48 hours | Liquid | Liquid | Liquid | Liquid |
| | | 50°C, 1 hour | Liquid | Liquid | Liquid | Liquid |
| | | 80°C, 1 hour | Liquid | Liquid | Liquid | Liquid |
| | | 80°C, 3 hours | Solidification | Liquid | Solidification | Liquid |
| | | Evaluation | B | A | B | A |
| | Colorability | 25°C, 0 minutes | Light yellow | Light yellow | Colorless | Colorless |
| | | 25°C, 24 hours | Light yellow | Light yellow | Colorless | Colorless |
| | | 25°C, 48 hours | Light yellow | Light yellow | Colorless | Colorless |
| | | 50°C, 1 hour | Light yellow | Light yellow | Colorless | Colorless |
| | | 80°C, 1 hour | Light yellow | Light yellow | Colorless | Colorless |
| | | 80°C, 3 hours | Light yellow | Light yellow | Light yellow | Colorless |
| | | Evaluation | C | C | B | A |

Table 2 (continued)

| | | | Example 1-19 | Example 1-20 | Example 1-21 | Example 1-22 |
|---|---|---|---|---|---|---|
| 1,1-Dicyanoethylene-containing composition | | | 1-19 | 1-20 | 1-21 | 1-22 |
| Composition | 1,1-Dicyanoethylene [parts by mass] | 1,1-Dicyanoethylene | 100 | 100 | 100 | 100 |
| | Component (B) [parts by mass] | (B1) | 0 | 0 | 0 | 0 |
| | Component (A-1) [parts by mass] | (A11) | 0 | 0 | 0 | 0 |
| | | (A12) | 0 | 0 | 0 | 0 |
| | | (A13) | 0 | 0 | 0 | 0 |
| | | (A14) | 0.1 | 0.1 | 0 | 0 |
| | | (A15) | 0 | 0 | 1 | 1 |
| | Comparative component (A-1) [parts by mass] | (A'11) | 0 | 0 | 0 | 0 |
| | | (A'12) | 0 | 0 | 0 | 0 |
| | | (A'13) | 0 | 0 | 0 | 0 |
| | | (A'14) | 0 | 0 | 0 | 0 |
| | | (A'15) | 0 | 0 | 0 | 0 |
| | Other component [parts by mass] | Methanesulfonic acid | 0 | 0.01 | 0 | 0.01 |
| Evaluation | Storage stability | 25°C, 0 minutes | Liquid | Liquid | Liquid | Liquid |
| | | 25°C, 24 hours | Liquid | Liquid | Liquid | Liquid |
| | | 25°C, 48 hours | Liquid | Liquid | Liquid | Liquid |
| | | 50°C, 1 hour | Liquid | Liquid | Thickening | Liquid |
| | | 80°C, 1 hour | Liquid | Liquid | Thickening | Liquid |
| | | 80°C, 3 hours | Solidification | Liquid | Solidification | Thickening |
| | | Evaluation | B | A | C1 | B |
| | Colorability | 25°C, 0 minutes | Colorless | Colorless | Colorless | Colorless |
| | | 25°C, 24 hours | Colorless | Colorless | Colorless | Colorless |
| | | 25°C, 48 hours | Colorless | Colorless | Colorless | Colorless |
| | | 50°C, 1 hour | Colorless | Colorless | Colorless | Colorless |
| | | 80°C, 1 hour | Colorless | Colorless | Colorless | Colorless |
| | | 80°C, 3 hours | Light yellow | Colorless | Colorless | Colorless |
| | | Evaluation | B | A | A | A |

[Table 3]

Table 3

| | | | Comparative Example 1-1 | Comparative Example 1-2 | Comparative Example 1-3 | Comparative Example 1-4 |
|---|---|---|---|---|---|---|
| 1,1-Dicyanoethylene-containing composition | | | 1'-1 | 1'-2 | 1'-3 | 1'-4 |
| Composition | 1,1-Dicyanoethylene [parts by mass] | 1,1-Dicyanoethylene | 100 | 100 | 100 | 100 |
| | Component (B) [parts by mass] | (B1) | 0 | 0 | 0 | 0 |
| | | (B2) | 0 | 0 | 0 | 0 |
| | | (B3) | 0 | 0 | 0 | 0 |
| | | (B4) | 0 | 0 | 0 | 0 |
| | Component (A-1) [parts by mass] | (A11) | 0 | 0 | 0 | 0 |
| | | (A12) | 0 | 0 | 0 | 0 |
| | | (A13) | 0 | 0 | 0 | 0 |
| | | (A14) | 0 | 0 | 0 | 0 |
| | | (A15) | 0 | 0 | 0 | 0 |
| | Comparative component (A-1) [parts by mass] | (A'11) | 0 | 1 | 0 | 0 |
| | | (A'12) | 0 | 0 | 1 | 0 |
| | | (A'13) | 0 | 0 | 0 | 0.0001 |
| | | (A'14) | 0 | 0 | 0 | 0 |
| | | (A'15) | 0 | 0 | 0 | 0 |
| | Other component [parts by mass] | Methanesulfonic acid | 0 | 0 | 0 | 0 |
| Evaluation | Storage stability | 25°C, 0 minutes | Liquid | Liquid | Liquid | Liquid |
| | | 25°C, 24 hours | Liquid | Solidification | Liquid | Liquid |
| | | 25°C, 48 hours | Liquid | Solidification | Solidification | Solidification |
| | | 50°C, 1 hour | Thickening | Solidification | Solidification | Solidification |
| | | 80°C, 1 hour | Solidification | Solidification | Solidification | Solidification |
| | | 80°C, 3 hours | Solidification | Solidification | Solidification | Solidification |
| | | Evaluation | C2 | E | D | D |
| | Colorability | 25°C, 0 minutes | Colorless | Colorless | Yellow | Colorless |
| | | 25°C, 24 hours | Colorless | Light yellow | Yellow | Colorless |
| | | 25°C, 48 hours | Colorless | Light yellow | Yellow | Light yellow |
| | | 50°C, 1 hour | Colorless | Light yellow | Yellow | Light yellow |
| | | 80°C, 1 hour | Colorless | Light yellow | Yellow | Light yellow |
| | | 80°C, 3 hours | Colorless | Light yellow | Yellow | Light yellow |
| | | Evaluation | A | C | E | C |

Table 3 (continued)

| | | | Comparative Example 1-5 | Comparative Example 1-6 | Comparative Example 1-7 | Comparative Example 1-8 |
|---|---|---|---|---|---|---|
| 1,1-Dicyanoethylene-containing composition | | | 1'-5 | 1'-6 | 1'-7 | 1'-8 |
| Composition | 1,1-Dicyanoethylene [parts by mass] | 1,1-Dicyanoethylene | 100 | 100 | 50 | 100 |
| | Component (B) [parts by mass] | (B1) | 0 | 0 | 50 | 0 |
| | | (B2) | 0 | 0 | 0 | 0 |
| | | (B3) | 0 | 0 | 0 | 0 |
| | | (B4) | 0 | 0 | 0 | 0 |
| | Component (A-1) [parts by mass] | (A11) | 0 | 0 | 0 | 0 |
| | | (A12) | 0 | 0 | 0 | 0 |
| | | (A13) | 0 | 0 | 0 | 0 |
| | | (A14) | 0 | 0 | 0 | 0 |
| | | (A15) | 0 | 0 | 0 | 0 |
| | Comparative component (A-1) [parts by mass] | (A'11) | 0 | 0 | 0 | 0 |
| | | (A'12) | 0 | 0 | 0 | 0 |
| | | (A'13) | 0.01 | 1 | 0.01 | 0 |
| | | (A'14) | 0 | 0 | 0 | 1 |
| | | (A'15) | 0 | 0 | 0 | 0 |
| | Other component [parts by mass] | Methanesulfonic acid | 0 | 0 | 0 | 0 |
| Evaluation | Storage stability | 25°C, 0 minutes | Liquid | Liquid | Liquid | Liquid |
| | | 25°C, 24 hours | Liquid | Liquid | Liquid | Liquid |
| | | 25°C, 48 hours | Solidification | Solidification | Solidification | Liquid |
| | | 50°C, 1 hour | Solidification | Solidification | Solidification | Solidification |
| | | 80°C, 1 hour | Solidification | Solidification | Solidification | Solidification |
| | | 80°C, 3 hours | Solidification | Solidification | Solidification | Solidification |
| | | Evaluation | D | D | D | C3 |
| | Colorability | 25°C, 0 minutes | Colorless | Colorless | Colorless | Colorless |
| | | 25°C, 24 hours | Colorless | Colorless | Colorless | Colorless |
| | | 25°C, 48 hours | Light yellow | Yellow | Yellow | Colorless |
| | | 50°C, 1 hour | Light yellow | Yellow | Yellow | Colorless |
| | | 80°C, 1 hour | Light yellow | Yellow | Yellow | Colorless |
| | | 80°C, 3 hours | Light yellow | Yellow | Yellow | Colorless |
| | | Evaluation | C | D | D | A |

Table 3 (continued)

| | | | Comparative Example 1-9 | Comparative Example 1-10 | Comparative Example 1-11 | Comparative Example 1-12 |
|---|---|---|---|---|---|---|
| 1,1-Dicyanoethylene-containing composition | | | 1'-9 | 1'-10 | 1'-11 | 1'-12 |
| Composition | 1,1-Dicyanoethylene [parts by mass] | 1,1-Dicyanoethylene | 100 | 50 | 50 | 50 |
| | Component (B) [parts by mass] | (B1) | 0 | 0 | 0 | 0 |
| | | (B2) | 0 | 50 | 0 | 0 |
| | | (B3) | 0 | 0 | 50 | 0 |
| | | (B4) | 0 | 0 | 0 | 50 |
| | Component (A-1) [parts by mass] | (A11) | 0 | 0 | 0 | 0 |
| | | (A12) | 0 | 0 | 0 | 0 |
| | | (A13) | 0 | 0 | 0 | 0 |
| | | (A14) | 0 | 0 | 0 | 0 |
| | | (A15) | 0 | 0 | 0 | 0 |
| | Comparative component (A-1) [parts by mass] | (A'11) | 0 | 0 | 0 | 0 |
| | | (A'12) | 0 | 0 | 0 | 0 |
| | | (A'13) | 0 | 0.01 | 0.01 | 0.01 |
| | | (A'14) | 0 | 0 | 0 | 0 |
| | | (A'15) | 1 | 0 | 0 | 0 |
| | Other component [parts by mass] | Methanesulfonic acid | 0 | 0 | 0 | 0 |
| Evaluation | Storage stability | 25°C, 0 minutes | Liquid | Liquid | Liquid | Liquid |
| | | 25°C, 24 hours | Liquid | Liquid | Liquid | Liquid |
| | | 25°C, 48 hours | Liquid | Liquid | Solidification | Liquid |
| | | 50°C, 1 hour | Solidification | Thickening | Solidification | Thickening |
| | | 80°C, 1 hour | Solidification | Solidification | Solidification | Solidification |
| | | 80°C, 3 hours | Solidification | Solidification | Solidification | Solidification |
| | | Evaluation | C3 | C2 | D | C2 |
| | Colorability | 25°C, 0 minutes | Colorless | Colorless | Colorless | Colorless |
| | | 25°C, 24 hours | Yellow | Colorless | Colorless | Colorless |
| | | 25°C, 48 hours | Yellow | Yellow | Yellow | Yellow |
| | | 50°C, 1 hour | Yellow | Yellow | Yellow | Yellow |
| | | 80°C, 1 hour | Yellow | Yellow | Yellow | Yellow |
| | | 80°C, 3 hours | Yellow | Yellow | Yellow | Yellow |
| | | Evaluation | E | D | D | D |

From Tables 1 to 3, it was found that the 1,1-dicyanoethylene-containing compositions according to Examples 1-1 to 1-22 were excellent in storage stability as compared with the 1,1-dicyanoethylene-containing compositions according to Comparative Examples 1-1 to 1-12. Furthermore, from Comparative Examples 1-2 and 1-3, it was found that the known radical polymerization inhibitors N-nitroso-N-phenylhydroxylamine aluminum and p-benzoquinone contribute little to the storage stability of the 1,1-dicyanoethylene-containing composition. According to the First Examples, since a 1,1-dicyanoethylene-containing composition having excellent storage stability can be provided, it has been found that even in a cured product and a laminate thereof, it can be expected to be able to suppress a decrease in physical properties due to the 1,1-dicyanoethylene-containing composition impairing storage stability.

[Second Examples]

[Component]

The components used in Examples and Comparative Examples were as follows.

<1,1-Dicyanoethylene>

1,1-Dicyanoethylene: 1,1-dicyanoethylene prepared in the First Examples

<Component (B): Polymerizable Monomer>

(B1): Ethyl 2-cyanoacrylate (manufactured by Sigma-Aldrich Co., LLC)
(B2): Methyl methacrylate (manufactured by Tokyo Chemical Industry Co., Ltd. (purity > 99.8%))
(B3): Styrene (manufactured by Tokyo Chemical Industry Co., Ltd. (purity > 99.0%))
(B4): Vinyl acetate (manufactured by Tokyo Chemical Industry Co., Ltd. (purity > 99.0%))

<Component (A-2): Phenol Compound>

(A21): Hydroquinone (manufactured by Tokyo Chemical Industry Co., Ltd.)
(A22): p-Methylphenol (manufactured by Tokyo Chemical Industry Co., Ltd.)
(A23): p-Methoxyphenol (manufactured by Tokyo Chemical Industry Co., Ltd.)
(A24): 2,6-Di-tert-butyl-4-methylphenol (manufactured by FUJIFILM Wako Pure Chemical Corporation)
(A25): 2,6-Di-tert-butylphenol (manufactured by Tokyo Chemical Industry Co., Ltd.)
(A26): "Irganox (registered trademark) 1010" manufactured by BASF SE
(A27): "Irganox (registered trademark) 1076" manufactured by FUJIFILM Wako Pure Chemical Corporation
(A28): 2,4-Di-tert-butylphenol (manufactured by Tokyo Chemical Industry Co., Ltd.)

Note that the component (A21), the component (A22), and the component (A23) were identical to the component (A'13), the component (A'14), and the component (A'15), respectively, in the First Examples, and the component (A24), the component (A25), the component (A26), the component (A27), and the component (A28) were identical to the component (A11), the component (A12), the component (A13), the component (A14), and the component (A15), respectively, in the First Examples.

<Component (C): Compound Having Specific Epa and Specific pKa>

(C1): Methanesulfonic acid (manufactured by Tokyo Chemical Industry Co., Ltd.)

Regarding this methanesulfonic acid, its Epa was 761.3 kJ/mol according to the literature value (Literature: NPL 2), and its pKa in the form of aqueous solution was -2.6 according to the literature value (Literature: NPL 3). Furthermore, the same methanesulfonic acid used as the other component in the First Examples was used as the component (C).

(C2): Sulfuric acid (manufactured by FUJIFILM Wako Pure Chemical Corporation)
Regarding this sulfuric acid, its Epa was 699.4 kJ/mol according to the literature value (Literature: NPL 2), and its pKa in the form of aqueous solution is -3.0 according to the literature value (Literature: NPL 3).

<Component (C')>

(C'1): Acetic acid (manufactured by KANTO CHEMICAL CO., INC.)
Regarding this acetic acid, its Epa was 783.7 kJ/mol according to the literature value (Literature: NPL 2), and its pKa in the form of aqueous solution was 4.8 according to the literature value (Literature: NPL 3).

[Evaluation Methods]

The liquid 1,1-dicyanoethylene-containing compositions obtained in Examples and Comparative Examples were used as samples. Their storage stability and colorability were evaluated using the same methods as in the First Examples.

<Example 2-1>

Under nitrogen atmosphere, a 1,1-dicyanoethylene-containing composition was prepared by mixing 100 parts by mass of 1,1-dicyanoethylene with 1 part by mass of hydroquinone (component (A22)) and 0.01 parts by mass of methanesulfonic acid (component (C)).

Using the prepared 1,1-dicyanoethylene-containing composition, the above-described evaluations of storage stability and colorability were carried out. The results are shown in Table 4.

<Examples 2-2 to 2-6 and 21 to 27>

1,1-Dicyanoethylene-containing compositions were prepared according to the formulations shown in Table 4. Multiple components were mixed simultaneously.

Using the prepared 1,1-dicyanoethylene-containing compositions, evaluations of storage stability and colorability were carried out in the same manner as in Example 2-1. The results are shown in Tables 4 and 6.

<Examples 2-7 to 2-20>

Examples 2-7 to 2-20 are identical to Examples 1-5 to 1-14, Example 1-16, Example 1-18, Example 1-20, and Example 1-22 shown in the First Examples, respectively, and are shown in Tables 5 and 6 along with their evaluation results.

<Comparative Examples 2-1 to 2-7 and 2-12 to 2-14>

Comparative Examples 2-1 to 2-7 and 2-12 to 2-14 are identical to Comparative Example 1-1 and Comparative Examples 1-4 to 1-12 shown in the First Examples, respectively, and are shown in Tables 7 and 8 along with their evaluation results.

<Comparative Examples 2-8 to 2-11>

1,1-Dicyanoethylene-containing compositions were prepared according to the formulations shown in Table 8. Multiple components were mixed simultaneously.

Using the prepared 1,1-dicyanoethylene-containing compositions, evaluations of storage stability and colorability were carried out in the same manner as in Example 2-1. The results are shown in Table 8.

[Table 4]

Table 4

| | | | Example 2-1 | Example 2-2 | Example 2-3 |
|---|---|---|---|---|---|
| 1,1-Dicyanoethylene-containing composition | | | 2-1 | 2-2 | 2-3 |
| Composition | 1,1-Dicyanoethylene [parts by mass] | 1,1-Dicyanoethylene | 100 | 100 | 100 |
| | Component (B) [parts by mass] | (B1) | 0 | 0 | 0 |
| | | (B2) | 0 | 0 | 0 |
| | | (B3) | 0 | 0 | 0 |
| | | (B4) | 0 | 0 | 0 |
| | Component (A-2) [parts by mass] | (A21) | 0 | 0 | 0 |
| | | (A22) | 1 | 0.01 | 0 |
| | | (A23) | 0 | 0 | 1 |
| | | (A24) | 0 | 0 | 0 |
| | | (A25) | 0 | 0 | 0 |
| | | (A26) | 0 | 0 | 0 |
| | | (A27) | 0 | 0 | 0 |
| | | (A28) | 0 | 0 | 0 |
| | Component (C) [parts by mass] | (C1) | 0.01 | 0.01 | 0.01 |
| | | (C2) | 0 | 0 | 0 |
| | Comparative component (C) [parts by mass] | (C'1) | 0 | 0 | 0 |
| Evaluation | Storage stability | 25°C, 0 minutes | Liquid | Liquid | Liquid |
| | | 25°C, 24 hours | Liquid | Liquid | Liquid |
| | | 25°C, 48 hours | Liquid | Liquid | Liquid |
| | | 50°C, 1 hour | Thickening | Liquid | Thickening |
| | | 80°C, 1 hour | Thickening | Liquid | Thickening |
| | | 80°C, 3 hours | Solidification | Solidification | Solidification |
| | | Evaluation | C1 | B | C1 |
| | Colorability | 25°C, 0 minutes | Colorless | Colorless | Colorless |
| | | 25°C, 24 hours | Colorless | Light yellow | Yellow |
| | | 25°C, 48 hours | Colorless | Light yellow | Yellow |
| | | 50°C, 1 hour | Colorless | Light yellow | Yellow |
| | | 80°C, 1 hour | Colorless | Light yellow | Yellow |
| | | 80°C, 3 hours | Colorless | Light yellow | Yellow |
| | | Evaluation | A | C | E |

Table 4 (continued)

| | | | Example 2-4 | Example 2-5 | Example 2-6 |
|---|---|---|---|---|---|
| 1,1-Dicyanoethylene-containing composition | | | 2-4 | 2-5 | 2-6 |
| Composition | 1,1-Dicyanoethylene [parts by mass] | 1,1-Dicyanoethylene | 100 | 100 | 50 |
| | Component (B) [parts by mass] | (B1) | 0 | 0 | 50 |
| | | (B2) | 0 | 0 | 0 |
| | | (B3) | 0 | 0 | 0 |
| | | (B4) | 0 | 0 | 0 |
| | Component (A-2) [parts by mass] | (A21) | 0 | 0.01 | 0.01 |
| | | (A22) | 0 | 0 | 0 |
| | | (A23) | 0.01 | 0 | 0 |
| | | (A24) | 0 | 0 | 0 |
| | | (A25) | 0 | 0 | 0 |
| | | (A26) | 0 | 0 | 0 |
| | | (A27) | 0 | 0 | 0 |
| | | (A28) | 0 | 0 | 0 |
| | Component (C) [parts by mass] | (C1) | 0.01 | 0.01 | 0.01 |
| | | (C2) | 0 | 0 | 0 |
| | Comparative component (C) [parts by mass] | (C'1) | 0 | 0 | 0 |
| Evaluation | Storage stability | 25°C, 0 minutes | Liquid | Liquid | Liquid |
| | | 25°C, 24 hours | Liquid | Liquid | Liquid |
| | | 25°C, 48 hours | Liquid | Liquid | Liquid |
| | | 50°C, 1 hour | Liquid | Liquid | Liquid |
| | | 80°C, 1 hour | Liquid | Liquid | Liquid |
| | | 80°C, 3 hours | Solidification | Thickening | Thickening |
| | | Evaluation | B | B | B |
| | Colorability | 25°C, 0 minutes | Colorless | Colorless | Colorless |
| | | 25°C, 24 hours | Light yellow | Colorless | Colorless |
| | | 25°C, 48 hours | Light yellow | Colorless | Colorless |
| | | 50°C, 1 hour | Light yellow | Light yellow | Light yellow |
| | | 80°C, 1 hour | Light yellow | Light yellow | Light yellow |
| | | 80°C, 3 hours | Light yellow | Light yellow | Light yellow |
| | | Evaluation | C | B | B |

[Table 5]

Table 5

| | | | Example 2-7 | Example 2-8 | Example 2-9 | Example 2-10 |
|---|---|---|---|---|---|---|
| 1,1-Dicyanoethylene-containing composition | | | 2-7 | 2-8 | 2-9 | 2-10 |
| Composition | 1,1-Dicyanoethylene [parts by mass] | 1,1-Dicyanoethylene | 100 | 100 | 100 | 100 |
| | Component (B) [parts by mass] | (B1) | 0 | 0 | 0 | 0 |
| | | (B2) | 0 | 0 | 0 | 0 |
| | | (B3) | 0 | 0 | 0 | 0 |
| | | (B4) | 0 | 0 | 0 | 0 |
| | Component (A-2) [parts by mass] | (A21) | 0 | 0 | 0 | 0 |
| | | (A22) | 0 | 0 | 0 | 0 |
| | | (A23) | 0 | 0 | 0 | 0 |
| | | (A24) | 1 | 1 | 0.1 | 0.01 |
| | | (A25) | 0 | 0 | 0 | 0 |
| | | (A26) | 0 | 0 | 0 | 0 |
| | | (A27) | 0 | 0 | 0 | 0 |
| | | (A28) | 0 | 0 | 0 | 0 |
| | Component (C) [parts by mass] | (C1) | 0.01 | 0.1 | 0.01 | 0.01 |
| | | (C2) | 0 | 0 | 0 | 0 |
| | Comparative component (C) [parts by mass] | (C'1) | 0 | 0 | 0 | 0 |
| Evaluation | Storage stability | 25°C, 0 minutes | Liquid | Liquid | Liquid | Liquid |
| | | 25°C, 24 hours | Liquid | Liquid | Liquid | Liquid |
| | | 25°C, 48 hours | Liquid | Liquid | Liquid | Liquid |
| | | 50°C, 1 hour | Liquid | Liquid | Liquid | Liquid |
| | | 80°C, 1 hour | Liquid | Liquid | Liquid | Liquid |
| | | 80°C, 3 hours | Liquid | Liquid | Liquid | Liquid |
| | | Evaluation | A | A | A | A |
| | Colorability | 25°C, 0 minutes | Colorless | Colorless | Colorless | Colorless |
| | | 25°C, 24 hours | Colorless | Colorless | Colorless | Colorless |
| | | 25°C, 48 hours | Colorless | Colorless | Colorless | Colorless |
| | | 50°C, 1 hour | Colorless | Colorless | Colorless | Colorless |
| | | 80°C, 1 hour | Colorless | Colorless | Colorless | Colorless |
| | | 80°C, 3 hours | Colorless | Colorless | Colorless | Colorless |
| | | Evaluation | A | A | A | A |

Table 5 (continued)

| | | | Example 2-11 | Example 2-12 | Example 2-13 | Example 2-14 |
|---|---|---|---|---|---|---|
| 1,1-Dicyanoethylene-containing composition | | | 2-11 | 2-12 | 2-13 | 2-14 |
| Composition | 1,1-Dicyanoethylene [parts by mass] | 1,1-Dicyanoethylene | 90 | 50 | 30 | 100 |
| | Component (B) [parts by mass] | (B1) | 10 | 50 | 70 | 0 |
| | | (B2) | 0 | 0 | 0 | 0 |
| | | (B3) | 0 | 0 | 0 | 0 |
| | | (B4) | 0 | 0 | 0 | 0 |
| | Component (A-2) [parts by mass] | (A21) | 0 | 0 | 0 | 0 |
| | | (A22) | 0 | 0 | 0 | 0 |
| | | (A23) | 0 | 0 | 0 | 0 |
| | | (A24) | 0.0001 | 0.0001 | 0.0001 | 0 |
| | | (A25) | 0 | 0 | 0 | 0.1 |
| | | (A26) | 0 | 0 | 0 | 0 |
| | | (A27) | 0 | 0 | 0 | 0 |
| | | (A28) | 0 | 0 | 0 | 0 |
| | Component (C) [parts by mass] | (C1) | 0.01 | 0.01 | 0.01 | 0.01 |
| | | (C2) | 0 | 0 | 0 | 0 |
| | Comparative component (C) [parts by mass] | (C'1) | 0 | 0 | 0 | 0 |
| Evaluation | Storage stability | 25°C, 0 minutes | Liquid | Liquid | Liquid | Liquid |
| | | 25°C, 24 hours | Liquid | Liquid | Liquid | Liquid |
| | | 25°C, 48 hours | Liquid | Liquid | Liquid | Liquid |
| | | 50°C, 1 hour | Liquid | Liquid | Liquid | Liquid |
| | | 80°C, 1 hour | Liquid | Liquid | Liquid | Liquid |
| | | 80°C, 3 hours | Liquid | Liquid | Liquid | Liquid |
| | | Evaluation | A | A | A | A |
| | Colorability | 25°C, 0 minutes | Colorless | Colorless | Colorless | Light yellow |
| | | 25°C, 24 hours | Colorless | Colorless | Colorless | Light yellow |
| | | 25°C, 48 hours | Colorless | Colorless | Colorless | Light yellow |
| | | 50°C, 1 hour | Colorless | Colorless | Colorless | Light yellow |
| | | 80°C, 1 hour | Colorless | Colorless | Colorless | Light yellow |
| | | 80°C, 3 hours | Colorless | Colorless | Colorless | Light yellow |
| | | Evaluation | A | A | A | C |

Table 5 (continued)

| | | | Example 2-15 | Example 2-16 | Example 2-17 |
|---|---|---|---|---|---|
| 1,1-Dicyanoethylene-containing composition | | | 2-15 | 2-16 | 2-17 |
| Composition | 1,1-Dicyanoethylene [parts by mass] | 1,1-Dicyanoethylene | 100 | 100 | 100 |
| | Component (B) [parts by mass] | (B1) | 0 | 0 | 0 |
| | | (B2) | 0 | 0 | 0 |
| | | (B3) | 0 | 0 | 0 |
| | | (B4) | 0 | 0 | 0 |
| | Component (A-2) [parts by mass] | (A21) | 0 | 0 | 0 |
| | | (A22) | 0 | 0 | 0 |
| | | (A23) | 0 | 0 | 0 |
| | | (A24) | 0 | 0 | 0 |
| | | (A25) | 0 | 0 | 0 |
| | | (A26) | 0.1 | 0 | 0 |
| | | (A27) | 0 | 0.1 | 0 |
| | | (A28) | 0 | 0 | 1 |
| | Component (C) [parts by mass] | (C1) | 0.01 | 0.01 | 0.01 |
| | | (C2) | 0 | 0 | 0 |
| | Comparative component (C) [parts by mass] | (C'1) | 0 | 0 | 0 |
| Evaluation | Storage stability | 25°C, 0 minutes | Liquid | Liquid | Liquid |
| | | 25°C, 24 hours | Liquid | Liquid | Liquid |
| | | 25°C, 48 hours | Liquid | Liquid | Liquid |
| | | 50°C, 1 hour | Liquid | Liquid | Liquid |
| | | 80°C, 1 hour | Liquid | Liquid | Liquid |
| | | 80°C, 3 hours | Liquid | Liquid | Thickening |
| | | Evaluation | A | A | B |
| | Colorability | 25°C, 0 minutes | Colorless | Colorless | Colorless |
| | | 25°C, 24 hours | Colorless | Colorless | Colorless |
| | | 25°C, 48 hours | Colorless | Colorless | Colorless |
| | | 50°C, 1 hour | Colorless | Colorless | Colorless |
| | | 80°C, 1 hour | Colorless | Colorless | Colorless |
| | | 80°C, 3 hours | Colorless | Colorless | Colorless |
| | | Evaluation | A | A | A |

[Table 6]

Table 6

| | | | Example 2-18 | Example 2-19 | Example 2-20 | Example 2-21 |
|---|---|---|---|---|---|---|
| 1,1-Dicyanoethylene-containing composition | | | 2-18 | 2-19 | 2-20 | 2-21 |
| Composition | 1,1-Dicyanoethylene [parts by mass] | 1,1-Dicyanoethylene | 50 | 50 | 50 | 50 |
| | Component (B) [parts by mass] | (B1) | 0 | 0 | 0 | 0 |
| | | (B2) | 50 | 0 | 0 | 50 |
| | | (B3) | 0 | 50 | 0 | 0 |
| | | (B4) | 0 | 0 | 50 | 0 |
| | Component (A-2) [parts by mass] | (A21) | 0 | 0 | 0 | 0.01 |
| | | (A22) | 0 | 0 | 0 | 0 |
| | | (A23) | 0 | 0 | 0 | 0 |
| | | (A24) | 0.0001 | 0.0001 | 0.0001 | 0 |
| | | (A25) | 0 | 0 | 0 | 0 |
| | | (A26) | 0 | 0 | 0 | 0 |
| | | (A27) | 0 | 0 | 0 | 0 |
| | | (A28) | 0 | 0 | 0 | 0 |
| | Component (C) [parts by mass] | (C1) | 0.01 | 0.01 | 0.01 | 0.01 |
| | | (C2) | 0 | 0 | 0 | 0 |
| | Comparative component (C) [parts by mass] | (C'1) | 0 | 0 | 0 | 0 |
| Evaluation | Storage stability | 25°C, 0 minutes | Liquid | Liquid | Liquid | Liquid |
| | | 25°C, 24 hours | Liquid | Liquid | Liquid | Liquid |
| | | 25°C, 48 hours | Liquid | Liquid | Liquid | Liquid |
| | | 50°C, 1 hour | Liquid | Liquid | Liquid | Liquid |
| | | 80°C, 1 hour | Liquid | Thickening | Liquid | Liquid |
| | | 80°C, 3 hours | Liquid | Thickening | Liquid | Thickening |
| | | Evaluation | A | B | A | B |
| | Colorability | 25°C, 0 minutes | Colorless | Colorless | Colorless | Colorless |
| | | 25°C, 24 hours | Colorless | Colorless | Colorless | Colorless |
| | | 25°C, 48 hours | Colorless | Colorless | Colorless | Colorless |
| | | 50°C, 1 hour | Colorless | Colorless | Colorless | Colorless |
| | | 80°C, 1 hour | Colorless | Colorless | Colorless | Colorless |
| | | 80°C, 3 hours | Colorless | Colorless | Colorless | Colorless |
| | | Evaluation | A | A | A | A |

Table 6 (continued)

| | | | Example 2-22 | Example 2-23 | Example 2-24 |
|---|---|---|---|---|---|
| 1,1-Dicyanoethylene-containing composition | | | 2-22 | 2-23 | 2-24 |
| Composition | 1,1-Dicyanoethylene [parts by mass] | 1,1-Dicyanoethylene | 50 | 50 | 100 |
| | Component (B) [parts by mass] | (B1) | 0 | 0 | 0 |
| | | (B2) | 0 | 0 | 0 |
| | | (B3) | 50 | 0 | 0 |
| | | (B4) | 0 | 50 | 0 |
| | Component (A-2) [parts by mass] | (A21) | 0.01 | 0.01 | 0 |
| | | (A22) | 0 | 0 | 1 |
| | | (A23) | 0 | 0 | 0 |
| | | (A24) | 0 | 0 | 0 |
| | | (A25) | 0 | 0 | 0 |
| | | (A26) | 0 | 0 | 0 |
| | | (A27) | 0 | 0 | 0 |
| | | (A28) | 0 | 0 | 0 |
| | Component (C) [parts by mass] | (C1) | 0.01 | 0.01 | 0 |
| | | (C2) | 0 | 0 | 0.01 |
| | Comparative component (C) [parts by mass] | (C'1) | 0 | 0 | 0 |
| Evaluation | Storage stability | 25°C, 0 minutes | Liquid | Liquid | Liquid |
| | | 25°C, 24 hours | Liquid | Liquid | Liquid |
| | | 25°C, 48 hours | Liquid | Liquid | Liquid |
| | | 50°C, 1 hour | Liquid | Liquid | Thickening |
| | | 80°C, 1 hour | Thickening | Liquid | Thickening |
| | | 80°C, 3 hours | Solidification | Thickening | Solidification |
| | | Evaluation | C1 | B | C1 |
| | Colorability | 25°C, 0 minutes | Colorless | Colorless | Colorless |
| | | 25°C, 24 hours | Colorless | Colorless | Colorless |
| | | 25°C, 48 hours | Colorless | Colorless | Colorless |
| | | 50°C, 1 hour | Light yellow | Colorless | Colorless |
| | | 80°C, 1 hour | Light yellow | Colorless | Colorless |
| | | 80°C, 3 hours | Light yellow | Colorless | Colorless |
| | | Evaluation | B | A | A |

Table 6 (continued)

| | | | Example 2-25 | Example 2-26 | Example 2-27 |
|---|---|---|---|---|---|
| 1,1-Dicyanoethylene-containing composition | | | 2-25 | 2-26 | 2-27 |
| Composition | 1,1-Dicyanoethylene [parts by mass] | 1,1-Dicyanoethylene | 100 | 100 | 100 |
| | Component (B) [parts by mass] | (B1) | 0 | 0 | 0 |
| | | (B2) | 0 | 0 | 0 |
| | | (B3) | 0 | 0 | 0 |
| | | (B4) | 0 | 0 | 0 |
| | Component (A-2) [parts by mass] | (A21) | 0 | 0 | 0 |
| | | (A22) | 0.01 | 0 | 0 |
| | | (A23) | 0 | 0 | 0 |
| | | (A24) | 0 | 1 | 1 |
| | | (A25) | 0 | 0 | 0 |
| | | (A26) | 0 | 0 | 0 |
| | | (A27) | 0 | 0 | 0 |
| | | (A28) | 0 | 0 | 0 |
| | Component (C) [parts by mass] | (C1) | 0 | 0 | 0 |
| | | (C2) | 0.01 | 0.01 | 0.1 |
| | Comparative component (C) [parts by mass] | (C'1) | 0 | 0 | 0 |
| Evaluation | Storage stability | 25°C, 0 minutes | Liquid | Liquid | Liquid |
| | | 25°C, 24 hours | Liquid | Liquid | Liquid |
| | | 25°C, 48 hours | Liquid | Liquid | Liquid |
| | | 50°C, 1 hour | Liquid | Liquid | Liquid |
| | | 80°C, 1 hour | Liquid | Liquid | Liquid |
| | | 80°C, 3 hours | Solidification | Liquid | Liquid |
| | | Evaluation | B | A | A |
| | Colorability | 25°C, 0 minutes | Colorless | Colorless | Colorless |
| | | 25°C, 24 hours | Light yellow | Colorless | Colorless |
| | | 25°C, 48 hours | Light yellow | Colorless | Colorless |
| | | 50°C, 1 hour | Light yellow | Colorless | Colorless |
| | | 80°C, 1 hour | Light yellow | Colorless | Colorless |
| | | 80°C, 3 hours | Light yellow | Colorless | Colorless |
| | | Evaluation | C | A | A |

[Table 7]

Table 7

| | | | Comparative Example 2-1 | Comparative Example 2-2 | Comparative Example 2-3 | Comparative Example 2-4 |
|---|---|---|---|---|---|---|
| 1,1-Dicyanoethylene-containing composition | | | 2'-1 | 2'-2 | 2'-3 | 2'-4 |
| Composition | 1,1-Dicyanoethylene [parts by mass] | 1,1-Dicyanoethylene | 100 | 100 | 100 | 100 |
| | Component (B) [parts by mass] | (B1) | 0 | 0 | 0 | 0 |
| | | (B2) | 0 | 0 | 0 | 0 |
| | | (B3) | 0 | 0 | 0 | 0 |
| | | (B4) | 0 | 0 | 0 | 0 |
| | Component (A-2) [parts by mass] | (A21) | 0 | 0.0001 | 0.01 | 1 |
| | | (A22) | 0 | 0 | 0 | 0 |
| | | (A23) | 0 | 0 | 0 | 0 |
| | | (A24) | 0 | 0 | 0 | 0 |
| | | (A25) | 0 | 0 | 0 | 0 |
| | | (A26) | 0 | 0 | 0 | 0 |
| | | (A27) | 0 | 0 | 0 | 0 |
| | | (A28) | 0 | 0 | 0 | 0 |
| | Component (C) [parts by mass] | (C1) | 0 | 0 | 0 | 0 |
| | | (C2) | 0 | 0 | 0 | 0 |
| | Comparative component (C) [parts by mass] | (C'1) | 0 | 0 | 0 | 0 |
| Evaluation | Storage stability | 25°C, 0 minutes | Liquid | Liquid | Liquid | Liquid |
| | | 25°C, 24 hours | Liquid | Liquid | Liquid | Liquid |
| | | 25°C, 48 hours | Liquid | Solidification | Solidification | Solidification |
| | | 50°C, 1 hour | Thickening | Solidification | Solidification | Solidification |
| | | 80°C, 1 hour | Solidification | Solidification | Solidification | Solidification |
| | | 80°C, 3 hours | Solidification | Solidification | Solidification | Solidification |
| | | Evaluation | C2 | D | D | D |
| | Colorability | 25°C, 0 minutes | Colorless | Colorless | Colorless | Colorless |
| | | 25°C, 24 hours | Colorless | Colorless | Colorless | Colorless |
| | | 25°C, 48 hours | Colorless | Light yellow | Light yellow | Yellow |
| | | 50°C, 1 hour | Colorless | Light yellow | Light yellow | Yellow |
| | | 80°C, 1 hour | Colorless | Light yellow | Light yellow | Yellow |
| | | 80°C, 3 hours | Colorless | Light yellow | Light yellow | Yellow |
| | | Evaluation | A | C | C | D |

Table 7 (continued)

| | | | Comparative Example 2-5 | Comparative Example 2-6 | Comparative Example 2-7 |
|---|---|---|---|---|---|
| 1,1-Dicyanoethylene-containing composition | | | 2'-5 | 2'-6 | 2'-7 |
| Composition | 1,1-Dicyanoethylene [parts by mass] | 1,1-Dicyanoethylene | 50 | 100 | 100 |
| | Component (B) [parts by mass] | (B1) | 50 | 0 | 0 |
| | | (B2) | 0 | 0 | 0 |
| | | (B3) | 0 | 0 | 0 |
| | | (B4) | 0 | 0 | 0 |
| | Component (A-2) [parts by mass] | (A21) | 0.01 | 0 | 0 |
| | | (A22) | 0 | 1 | 0 |
| | | (A23) | 0 | 0 | 1 |
| | | (A24) | 0 | 0 | 0 |
| | | (A25) | 0 | 0 | 0 |
| | | (A26) | 0 | 0 | 0 |
| | | (A27) | 0 | 0 | 0 |
| | | (A28) | 0 | 0 | 0 |
| | Component (C) [parts by mass] | (C1) | 0 | 0 | 0 |
| | | (C2) | 0 | 0 | 0 |
| | Comparative component (C) [parts by mass] | (C'1) | 0 | 0 | 0 |
| Evaluation | Storage stability | 25°C, 0 minutes | Liquid | Liquid | Liquid |
| | | 25°C, 24 hours | Liquid | Liquid | Liquid |
| | | 25°C, 48 hours | Solidification | Liquid | Liquid |
| | | 50°C, 1 hour | Solidification | Solidification | Solidification |
| | | 80°C, 1 hour | Solidification | Solidification | Solidification |
| | | 80°C, 3 hours | Solidification | Solidification | Solidification |
| | | Evaluation | D | C3 | C3 |
| | Colorability | 25°C, 0 minutes | Colorless | Colorless | Colorless |
| | | 25°C, 24 hours | Colorless | Colorless | Yellow |
| | | 25°C, 48 hours | Yellow | Colorless | Yellow |
| | | 50°C, 1 hour | Yellow | Colorless | Yellow |
| | | 80°C, 1 hour | Yellow | Colorless | Yellow |
| | | 80°C, 3 hours | Yellow | Colorless | Yellow |
| | | Evaluation | D | A | E |

[Table 8]

Table 8

| | | | Comparative Example 2-8 | Comparative Example 2-9 | Comparative Example 2-10 | Comparative Example 2-11 |
|---|---|---|---|---|---|---|
| 1,1-Dicyanoethylene-containing composition | | | 2'-8 | 2'-9 | 2'-10 | 2'-11 |
| Composition | 1,1-Dicyanoethylene [parts by mass] | 1,1-Dicyanoethylene | 100 | 100 | 100 | 100 |
| | Component (B) [parts by mass] | (B1) | 0 | 0 | 0 | 0 |
| | | (B2) | 0 | 0 | 0 | 0 |
| | | (B3) | 0 | 0 | 0 | 0 |
| | | (B4) | 0 | 0 | 0 | 0 |
| | Component (A-2) [parts by mass] | (A21) | 0 | 0 | 0 | 0 |
| | | (A22) | 1 | 0.01 | 0 | 0 |
| | | (A23) | 0 | 0 | 0 | 0 |
| | | (A24) | 0 | 0 | 1 | 1 |
| | | (A25) | 0 | 0 | 0 | 0 |
| | | (A26) | 0 | 0 | 0 | 0 |
| | | (A27) | 0 | 0 | 0 | 0 |
| | | (A28) | 0 | 0 | 0 | 0 |
| | Component (C) [parts by mass] | (C1) | 0 | 0 | 0 | 0 |
| | | (C2) | 0 | 0 | 0 | 0 |
| | Comparative component (C) [parts by mass] | (C'1) | 0.01 | 0.01 | 0.01 | 0.1 |
| Evaluation | Storage stability | 25°C, 0 minutes | Liquid | Liquid | Liquid | Liquid |
| | | 25°C, 24 hours | Liquid | Liquid | Liquid | Liquid |
| | | 25°C, 48 hours | Liquid | Liquid | Liquid | Liquid |
| | | 50°C, 1 hour | Solidification | Solidification | Solidification | Solidification |
| | | 80°C, 1 hour | Solidification | Solidification | Solidification | Solidification |
| | | 80°C, 3 hours | Solidification | Solidification | Solidification | Solidification |
| | | Evaluation | C3 | C3 | C3 | C3 |
| | Colorability | 25°C, 0 minutes | Colorless | Colorless | Colorless | Colorless |
| | | 25°C, 24 hours | Colorless | Light yellow | Colorless | Colorless |
| | | 25°C, 48 hours | Colorless | Light yellow | Colorless | Colorless |
| | | 50°C, 1 hour | Colorless | Light yellow | Colorless | Colorless |
| | | 80°C, 1 hour | Colorless | Light yellow | Colorless | Colorless |
| | | 80°C, 3 hours | Colorless | Light yellow | Colorless | Colorless |
| | | Evaluation | A | C | A | A |

Table 8 (continued)

| | | | Comparative Example 2-12 | Comparative Example 2-13 | Comparative Example 2-14 |
|---|---|---|---|---|---|
| 1,1-Dicyanoethylene-containing composition | | | 2'-12 | 2'-13 | 2'-14 |
| Composition | 1,1-Dicyanoethylene [parts by mass] | 1,1-Dicyanoethylene | 50 | 50 | 50 |
| | Component (B) [parts by mass] | (B1) | 0 | 0 | 0 |
| | | (B2) | 50 | 0 | 0 |
| | | (B3) | 0 | 50 | 0 |
| | | (B4) | 0 | 0 | 50 |
| | Component (A-2) [parts by mass] | (A21) | 0.01 | 0.01 | 0.01 |
| | | (A22) | 0 | 0 | 0 |
| | | (A23) | 0 | 0 | 0 |
| | | (A24) | 0 | 0 | 0 |
| | | (A25) | 0 | 0 | 0 |
| | | (A26) | 0 | 0 | 0 |
| | | (A27) | 0 | 0 | 0 |
| | | (A28) | 0 | 0 | 0 |
| | Component (C) [parts by mass] | (C1) | 0 | 0 | 0 |
| | | (C2) | 0 | 0 | 0 |
| | Comparative component (C) [parts by mass] | (C'1) | 0 | 0 | 0 |
| Evaluation | Storage stability | 25°C, 0 minutes | Liquid | Liquid | Liquid |
| | | 25°C, 24 hours | Liquid | Liquid | Liquid |
| | | 25°C, 48 hours | Solidification | Solidification | Solidification |
| | | 50°C, 1 hour | Solidification | Solidification | Solidification |
| | | 80°C, 1 hour | Solidification | Solidification | Solidification |
| | | 80°C, 3 hours | Solidification | Solidification | Solidification |
| | | Evaluation | D | D | D |
| | Colorability | 25°C, 0 minutes | Colorless | Colorless | Colorless |
| | | 25°C, 24 hours | Colorless | Colorless | Colorless |
| | | 25°C, 48 hours | Yellow | Yellow | Yellow |
| | | 50°C, 1 hour | Yellow | Yellow | Yellow |
| | | 80°C, 1 hour | Yellow | Yellow | Yellow |
| | | 80°C, 3 hours | Yellow | Yellow | Yellow |
| | | Evaluation | D | D | D |

From Tables 4 to 8, it was found that the 1,1-dicyanoethylene-containing compositions according to Examples 2-1 to 2-27 were excellent in storage stability as compared with the 1,1-dicyanoethylene-containing compositions according to Comparative Examples 2-1 to 2-14. It was also found that in some of the embodiments corresponding to the comparative examples in the First Examples, by using the component (C) in combination, it is possible to enhance the storage stability of the 1,1-dicyanoethylene-containing composition. Furthermore, it was found that in the embodiments corresponding to the examples in the First Examples, by using the component (C) in combination, it is possible to further enhance either or both the storage stability and colorability of the 1,1-dicyanoethylene-containing composition.

According to the Second Examples, since a 1,1-dicyanoethylene-containing composition having excellent storage stability can be provided, it has been found that even in a cured product and a laminate thereof, it can be expected to be able to suppress a decrease in physical properties due to the 1,1-dicyanoethylene-containing composition impairing storage stability.

[Examples of Laminate]

[Adherend]

Steel plate (manufactured by Standard Test Piece Co., Ltd., height 25 mm, width 100 mm, thickness 1.6 mm)
Aluminum plate (manufactured by Standard Test Piece Co., Ltd., height 25 mm, width 100 mm, thickness 1.6 mm)
Rigid polyvinyl chloride (PVC) plate (manufactured by Nippon Testpanel Co., Ltd., height 25 mm, width 100 mm, thickness 2.0 mm)

[Measurement Methods]

The tensile shear adhesive strength was measured as follows using the laminates obtained in Examples and Comparative Examples as samples.

<Tensile Shear Adhesive Strength>

The tensile shear adhesive strength of the laminate was measured in an environment at a room temperature of 25°C and a humidity of 25% using a universal material tester Model 5969 (manufactured by Instron Corporation) at a tensile speed of 20 mm/min.

<Examples 3-1 to 3-10 and Comparative Examples 3-1 to 3-3>

A 1,1-dicyanoethylene-containing composition (100 μL) shown in Table 9 was applied to an area of 12.5 mm × 25 mm of an adherend (1) shown in Table 9, and an adherend (2) shown in Table 9 was stacked on the applied surface. Thereafter, the 1,1-dicyanoethylene-containing composition was allowed to stand for 1 day in an environment at a room temperature of 23°C and a humidity of 50% to cure the 1,1-dicyanoethylene-containing composition, thereby bonding the adherends (1) and (2) to obtain a laminate.
Using the obtained laminate, the tensile shear adhesive strength was measured. The results are shown in Table 9. In Example 3-3, the adherend failed, and it was not possible to measure the tensile shear adhesive strength.
[Table 9]

Table 9

| | Example 3-1 | Example 3-2 | Example 3-3 | Example 3-4 | Example 3-5 |
|---|---|---|---|---|---|
| 1,1-Dicyanoethylene-containing composition | 1-1 | 1-1 | 1-1 | 1-6 | 1-7 |
| Adherend (1) | Steel plate | Aluminum | PVC | Steel plate | Steel plate |
| Adherend (2) | Steel plate | Steel plate | PVC | Steel plate | Steel plate |
| Tensile shear adhesive strength [MPa] | 11.5 | 11.8 | - | 12.0 | 12.2 |

Table 9 (continued)

| | Example 3-6 | Example 3-7 | Example 3-8 | Example 3-9 |
|---|---|---|---|---|
| 1,1-Dicyanoethylene-containing composition | 1-8 | 1-9 | 1-10 | 2-7 |
| Adherend (1) | Steel plate | Steel plate | Steel plate | Steel plate |
| Adherend (2) | Steel plate | Steel plate | Steel plate | Steel plate |
| Tensile shear adhesive strength [MPa] | 12.0 | 12.3 | 13.0 | 12.8 |

Table 9 (continued)

| | Example 3-10 | Comparative Example 3-1 | Comparative Example 3-2 | Comparative Example 3-3 |
|---|---|---|---|---|
| 1,1-Dicyanoethylene-containing composition | 2-8 | 1'-1 | 1'-2 | 2'-6 |
| Adherend (1) | Steel plate | Steel plate | Steel plate | Steel plate |
| Adherend (2) | Steel plate | Steel plate | Steel plate | Steel plate |

(continued)

| | Example 3-10 | Comparative Example 3-1 | Comparative Example 3-2 | Comparative Example 3-3 |
|---|---|---|---|---|
| Tensile shear adhesive strength [MPa] | 12.4 | 3.0 | 2.5 | 2.2 |

Table 9 shows that the laminates according to Examples 3-1, 3-2, and 3-4 to 3-10 exhibit higher tensile shear adhesive strength than the laminates according to Comparative Examples 3-1 to 3-3. Specifically, it was found that the deterioration in physical properties caused by the loss of storage stability in the 1,1-dicyanoethylene-containing composition could be suppressed.

**Claims**

1. A 1,1-dicyanoethylene-containing composition comprising 1,1-dicyanoethylene and an o-substituted phenol compound (A-1) represented by the following formula (I):

[Chem. 1]

OH

$R^{15}$ $R^{11}$

$R^{14}$ $R^{12}$

$R^{13}$

(I)

wherein $R^{11}$ and $R^{15}$ are each independently a hydrogen atom or an o-substituent which is one selected from the group consisting of an alkyl group, a cycloalkyl group, an aryl group, an alkoxy group, a carboxy group, an ester group represented by $-COOR^{1a}$ (where $R^{1a}$ is an alkyl group having 1 to 25 carbon atoms), an acid anhydride group, an acyl group represented by $-COR^{1b}$ (where $R^{1b}$ is an alkyl group having 1 to 25 carbon atoms), an acyloxy group represented by $-OCOR^{1c}$ (where $R^{1c}$ is an alkyl group having 1 to 25 carbon atoms), a halogen atom, and a haloalkyl group (provided that a case in which both of $R^{11}$ and $R^{15}$ are hydrogen atoms is excluded);
$R^{13}$ is a hydrogen atom or a p-substituent, and the p-substituent is the same as the o-substituent; and
$R^{12}$ and $R^{14}$ are each independently a hydrogen atom or a m-substituent, and the m-substituent is the same as the p-substituent.

2. The 1,1-dicyanoethylene-containing composition according to claim 1, wherein one or both of $R^{11}$ and $R^{15}$ have a Taft steric parameter Es value of less than 1.24.

3. The 1,1-dicyanoethylene-containing composition according to claim 1, wherein one or both of $R^{11}$ and $R^{15}$ are each independently one selected from the group consisting of a branched alkyl group, a cycloalkyl group, and an aryl group.

4. The 1,1-dicyanoethylene-containing composition according to claim 1, wherein the o-substituted phenol compound (A-1) is at least one selected from 2,6-di-tert-butylphenol, 2,6-di-tert-butyl-4-methylphenol, pentaerythritol tetrakis [3-(3,5-di-tert-butyl-4-hydroxyphenyl)propionate], octadecyl 3-(3,5-di-tert-butyl-4-hydroxyphenyl)propionate, 2,4-di-tert-butylphenol, 1,1,3-tris(2-methyl-4-hydroxy-5-tert-butylphenyl)butane, and 4,4'-butylidenebis(6-tert-butyl-m-cresol).

5. The 1,1-dicyanoethylene-containing composition according to claim 1, wherein both of $R^{11}$ and $R^{15}$ have a Taft steric parameter Es value of less than 1.24.

6. The 1,1-dicyanoethylene-containing composition according to claim 1, wherein both of $R^{11}$ and $R^{15}$ are each independently one selected from the group consisting of a branched alkyl group, a cycloalkyl group, and an aryl group.

7. The 1,1-dicyanoethylene-containing composition according to claim 1, wherein the o-substituted phenol compound (A-1) comprises at least one selected from 2,6-di-tert-butylphenol, 2,6-di-tert-butyl-4-methylphenol, pentaerythritol tetrakis[3-(3,5-di-tert-butyl-4-hydroxyphenyl)propionate], and octadecyl 3-(3,5-di-tert-butyl-4-hydroxyphenyl)propionate.

8. The 1,1-dicyanoethylene-containing composition according to claim 1, excluding a case where both $R^{11}$ and $R^{15}$ are halogen atoms.

9. The 1,1-dicyanoethylene-containing composition according to any one of claims 1 to 8, further comprising a polymerizable monomer (B).

10. The 1,1-dicyanoethylene-containing composition according to claim 9, wherein the polymerizable monomer (B) is one or more selected from the group consisting of ethylene, propylene, butadiene, isobutylene, isoprene, 1-hexene, 1-octene, vinyl acetate, vinyl propionate, vinyl butyrate, styrene, $\alpha$-methylstyrene, p-methylstyrene, acrylic acid, methacrylic acid, alkyl acrylate, alkyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester.

11. The 1,1-dicyanoethylene-containing composition according to claim 10, wherein the polymerizable monomer (B) comprises 2-cyanoacrylic acid alkyl ester.

12. The 1,1-dicyanoethylene-containing composition according to claim 11, wherein the 2-cyanoacrylic acid alkyl ester is ethyl 2-cyanoacrylate.

13. A cured product obtained by reacting 1,1-dicyanoethylene with a Lewis basic compound in the presence of an o-substituted phenol compound (A-1) represented by the following formula (I):

[Chem. 2]

OH

$R^{15}$ $R^{11}$

$R^{14}$ $R^{12}$

$R^{13}$

(I)

wherein $R^{11}$ and $R^{15}$ are each independently a hydrogen atom or an o-substituent which is one selected from the group consisting of an alkyl group, a cycloalkyl group, an aryl group, an alkoxy group, a carboxy group, an ester group represented by $-COOR^{1a}$ (where $R^{1a}$ is an alkyl group having 1 to 25 carbon atoms), an acid anhydride group, an acyl group represented by $-COR^{1b}$ (where $R^{1b}$ is an alkyl group having 1 to 25 carbon atoms), an acyloxy group represented by $-OCOR^{1c}$ (where $R^{1c}$ is an alkyl group having 1 to 25 carbon atoms), a halogen atom, and a haloalkyl group (provided that a case in which both of $R^{11}$ and $R^{15}$ are hydrogen atoms is excluded);
$R^{13}$ is a hydrogen atom or a p-substituent, and the p-substituent is the same as the o-substituent; and
$R^{12}$ and $R^{14}$ are each independently a hydrogen atom or a m-substituent, and the m-substituent is the same as the p-substituent.

14. The cured product according to claim 13, wherein one or both of $R^{11}$ and $R^{15}$ have a Taft steric parameter Es value of less than 1.24.

15. The cured product according to claim 13, wherein one or both of $R^{11}$ and $R^{15}$ are each independently one selected from the group consisting of a branched alkyl group, a cycloalkyl group, and an aryl group.

16. The cured product according to claim 13, wherein the o-substituted phenol compound (A-1) is one selected from 2,6-di-tert-butylphenol, 2,6-di-tert-butyl-4-methylphenol, pentaerythritol tetrakis[3-(3,5-di-tert-butyl-4-hydroxyphenyl) propionate], octadecyl 3-(3,5-di-tert-butyl-4-hydroxyphenyl)propionate, 2,4-di-tert-butylphenol, 1,1,3-tris(2-

methyl-4-hydroxy-5-tert-butylphenyl)butane, and 4,4'-butylidenebis(6-tert-butyl-m-cresol).

**17.** The cured product according to claim 13, wherein both of $R^{11}$ and $R^{15}$ have a Taft steric parameter Es value of less than 1.24.

**18.** The cured product according to claim 13, wherein both of $R^{11}$ and $R^{15}$ are each independently one selected from the group consisting of a branched alkyl group, a cycloalkyl group, and an aryl group.

**19.** The cured product according to claim 13, wherein the o-substituted phenol compound (A-1) comprises at least one selected from 2,6-di-tert-butylphenol, 2,6-di-tert-butyl-4-methylphenol, pentaerythritol tetrakis[3-(3,5-di-tert-butyl-4-hydroxyphenyl)propionate], and octadecyl 3-(3,5-di-tert-butyl-4-hydroxyphenyl)propionate.

**20.** The cured product according to claim 13, excluding a case where both $R^{11}$ and $R^{15}$ are halogen atoms.

**21.** The cured product according to claim 13, which is obtained by reacting 1,1-dicyanoethylene, a polymerizable monomer (B), and the Lewis basic compound.

**22.** The cured product according to claim 21, wherein the polymerizable monomer (B) is one or more selected from the group consisting of ethylene, propylene, butadiene, isobutylene, isoprene, 1-hexene, 1-octene, vinyl acetate, vinyl propionate, vinyl butyrate, styrene, α-methylstyrene, p-methylstyrene, acrylic acid, methacrylic acid, alkyl acrylate, alkyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester.

**23.** The cured product according to claim 22, wherein the polymerizable monomer (B) comprises 2-cyanoacrylic acid alkyl ester.

**24.** The cured product according to claim 23, wherein the 2-cyanoacrylic acid alkyl ester is ethyl 2-cyanoacrylate.

**25.** A laminate comprising the cured product according to any one of claims 13 to 24 and an adherend bonded to the cured product.

**26.** A 1,1-dicyanoethylene-containing composition comprising 1,1-dicyanoethylene, a phenol compound (A-2) represented by the following formula (II), and a compound (C) having a proton affinity (Epa) of 780 kJ/mol or less and a pKa of 5 or less in the form of an aqueous solution:

[Chem. 3]

OH

$R^{25}$ $R^{21}$

$R^{24}$ $R^{22}$

$R^{23}$

(II)

wherein $R^{21}$ and $R^{25}$ are each independently a hydrogen atom or an o-substituent which is one selected from the group consisting of an alkyl group, a cycloalkyl group, an aryl group, an alkoxy group, a carboxy group, an ester group represented by $-COOR^{2a}$ (where $R^{2a}$ is an alkyl group having 1 to 25 carbon atoms), an acid anhydride group, an acyl group represented by $-COR^{2b}$ (where $R^{2b}$ is a hydrogen atom or an alkyl group having 1 to 25 carbon atoms), an acyloxy group represented by $-OCOR^{2c}$ (where $R^{2c}$ is an alkyl group having 1 to 25 carbon atoms), a hydroxy group, an amino group, a halogen atom, and a haloalkyl group;
$R^{23}$ is a hydrogen atom or a p-substituent, and the p-substituent is the same as the o-substituent; and
$R^{22}$ and $R^{24}$ are each independently a hydrogen atom or a m-substituent, and the m-substituent is the same as the p-substituent.

**27.** The 1,1-dicyanoethylene-containing composition according to claim 26, wherein both $R^{21}$ and $R^{25}$ are hydrogen atoms.

**28.** The 1,1-dicyanoethylene-containing composition according to claim 26, wherein the compound (C) is at least one selected from the group consisting of methanesulfonic acid, sulfuric acid, and sulfur dioxide.

**29.** The 1,1-dicyanoethylene-containing composition according to any one of claims 26 to 28, further comprising a polymerizable monomer (B).

**30.** The 1,1-dicyanoethylene-containing composition according to claim 29, wherein the polymerizable monomer (B) is one or more selected from the group consisting of ethylene, propylene, butadiene, isobutylene, isoprene, 1-hexene, 1-octene, vinyl acetate, styrene, methyl methacrylate, dodecyl methacrylate, 2-cyanoacrylic acid alkyl ester, 2-cyanopentadienoic acid alkyl ester, and methylidenemalonic acid dialkyl ester.

**31.** The 1,1-dicyanoethylene-containing composition according to claim 30, wherein the polymerizable monomer (B) comprises 2-cyanoacrylic acid alkyl ester.

**32.** The 1,1-dicyanoethylene-containing composition according to claim 31, wherein the 2-cyanoacrylic acid alkyl ester is ethyl 2-cyanoacrylate.

**33.** A cured product obtained by reacting 1,1-dicyanoethylene with a Lewis basic compound in the presence of a phenol compound (A-2) represented by the following formula (II) and a compound (C) having a proton affinity (Epa) of 780 kJ/mol or less and a pKa of 5 or less in the form of an aqueous solution:

[Chem. 4]

OH
$R^{25}$ $R^{21}$
$R^{24}$ $R^{22}$
$R^{23}$

(II)

wherein $R^{21}$ and $R^{25}$ are each independently a hydrogen atom or an o-substituent which is one selected from the group consisting of an alkyl group, a cycloalkyl group, an aryl group, an alkoxy group, a carboxy group, an ester group represented by $-COOR^{2a}$ (where $R^{2a}$ is an alkyl group having 1 to 25 carbon atoms), an acid anhydride group, an acyl group represented by $-COR^{2b}$ (where $R^{2b}$ is a hydrogen atom or an alkyl group having 1 to 25 carbon atoms), an acyloxy group represented by $-OCOR^{2c}$ (where $R^{2c}$ is an alkyl group having 1 to 25 carbon atoms), a hydroxy group, an amino group, a halogen atom, and a haloalkyl group;
$R^{23}$ is each independently a hydrogen atom or a p-substituent, and the p-substituent is the same as the o-substituent; and
$R^{22}$ and $R^{24}$ are each independently a hydrogen atom or a m-substituent, and the m-substituent is the same as the p-substituent.

**34.** The cured product according to claim 33, wherein both $R^{21}$ and $R^{25}$ are hydrogen atoms.

**35.** The cured product according to claim 33, wherein the compound (C) is at least one selected from the group consisting of methanesulfonic acid, sulfuric acid, and sulfur dioxide.

**36.** The cured product according to claim 33, which is obtained by reacting 1,1-dicyanoethylene, a polymerizable monomer (B), and the Lewis basic compound.

**37.** The cured product according to claim 36, wherein the polymerizable monomer (B) is one or more selected from the group consisting of ethylene, propylene, butadiene, isobutylene, isoprene, 1-hexene, 1-octene, vinyl acetate, vinyl propionate, vinyl butyrate, styrene, α-methylstyrene, p-methylstyrene, acrylic acid, methacrylic acid, alkyl acrylate, alkyl methacrylate, acrylonitrile, vinyl chloride, vinylidene chloride, vinylidene fluoride, 2-cyanoacrylic acid alkyl ester, 2-cyanopentadienoic acid alkyl ester, and methylidenemalonic acid dialkyl ester.

**38.** The cured product according to claim 37, wherein the polymerizable monomer (B) comprises 2-cyanoacrylic acid alkyl ester.

**39.** The cured product according to claim 38, wherein the 2-cyanoacrylic acid alkyl ester is ethyl 2-cyanoacrylate.

**40.** A laminate comprising the cured product according to any one of claims 33 to 39 and an adherend bonded to the cured product.

## INTERNATIONAL SEARCH REPORT

International application No.
**PCT/JP2024/039898**

**A. CLASSIFICATION OF SUBJECT MATTER**

***C07C 253/32***(2006.01)i; ***C07C 39/06***(2006.01)i; ***C07C 39/07***(2006.01)i; ***C07C 39/08***(2006.01)i; ***C07C 43/23***(2006.01)i; ***C07C 69/732***(2006.01)i; ***C07C 255/09***(2006.01)i; ***C07C 255/23***(2006.01)i; ***C07C 309/04***(2006.01)i; ***C08F 2/40***(2006.01)i; ***C08F 2/44***(2006.01)i; ***C08F 22/30***(2006.01)i; ***C08F 22/34***(2006.01)i; ***C08K 5/13***(2006.01)i; ***C08L 35/04***(2006.01)i; ***C09J 4/00***(2006.01)i

FI: C07C253/32; C07C39/06; C07C39/07; C07C39/08; C07C43/23 Z; C07C69/732 Z; C07C255/09; C07C255/23; C07C309/04; C08F2/40; C08F2/44 B; C08F22/30; C08F22/34; C08K5/13; C08L35/04; C09J4/00

According to International Patent Classification (IPC) or to both national classification and IPC

**B. FIELDS SEARCHED**

Minimum documentation searched (classification system followed by classification symbols)

C07C253/32; C07C39/06; C07C39/07; C07C39/08; C07C43/23; C07C69/732; C07C255/09; C07C255/23; C07C309/04; C08F2/40; C08F2/44; C08F22/30; C08F22/34; C08K5/13; C08L35/04; C09J4/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 63-277218 A (TOPPAN PRINTING CO., LTD.) 15 November 1988 (1988-11-15)<br>claims, p. 2, upper right column to lower left column, p. 4, upper left column, example 2 | 1-40 |
| Y | JP 1-135754 A (BAYER AKTIENGESELLSCHAFT) 29 May 1989 (1989-05-29)<br>claims, p. 2, lower right column to p. 3, upper right column | 1-40 |
| Y | WO 2019/082601 A1 (SHIN-ETSU CHEMICAL CO., LTD.) 02 May 2019 (2019-05-02)<br>paragraph [0041] | 1-40 |
| A | JP 2008-174494 A (NIPPON SHOKUBAI CO., LTD.) 31 July 2008 (2008-07-31)<br>entire text | 1-40 |
| A | JP 2000-7999 A (CLOSURE MEDICAL CORPORATION) 11 January 2000 (2000-01-11)<br>entire text | 1-40 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 December 2024** | **04 February 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/039898**

**C. DOCUMENTS CONSIDERED TO BE RELEVANT**

| Category* | Citation of document, with indication, where appropriate, of the relevant passages | Relevant to claim No. |
|---|---|---|
| A | US 2467378 A (THE B. F. GOODRICH COMPANY) 19 April 1949 (1949-04-19) entire text | 1-40 |
| A | JP 2021-59620 A (RICOH COMPANY, LTD.) 15 April 2021 (2021-04-15) entire text | 1-40 |

Form PCT/ISA/210 (second sheet) (July 2022)

**INTERNATIONAL SEARCH REPORT**
**Information on patent family members**

International application No.
**PCT/JP2024/039898**

| Patent document cited in search report | Publication date (day/month/year) | Patent family member(s) | Publication date (day/month/year) |
|---|---|---|---|
| JP 63-277218 A | 15 November 1988 | (Family: none) | |
| JP 1-135754 A | 29 May 1989 | US 4986884 A<br>claims, columns 2-3<br>EP 267488 A1<br>DE 3638171 A1 | |
| WO 2019/082601 A1 | 02 May 2019 | US 2020/0339757 A1<br>paragraph [0062]<br>EP 3702396 A1<br>CN 111247193 A | |
| JP 2008-174494 A | 31 July 2008 | (Family: none) | |
| JP 2000-7999 A | 11 January 2000 | US 2002/0055573 A1<br>entire text<br>EP 965623 A1 | |
| US 2467378 A | 19 April 1949 | (Family: none) | |
| JP 2021-59620 A | 15 April 2021 | US 2021/0102081 A1<br>entire text | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2665298 A
- US 2535861 A
- JP 2008174494 A
- US 2476270 A


### Non-patent literature cited in the description


- *J. Am. Chem. Soc.*, 1989, vol. 111, 9078-9081
- *Tetrahedron*, 1978, vol. 34, 3553-3562
- **EDWARD P. H** ; **DHARON G. L**. *Journal of Physical and Chemical Reference Data*, 1998, vol. 27, 413-656